# EUROPÄISCHE PATENTSCHRIFT

(11) **EP 4 591 036 B1**
(45) Veröffentlichungstag und Bekanntmachung des Hinweises auf die Patenterteilung: **16.09.2026**
(21) Anmeldenummer: 23773286.2
(22) Anmeldetag: 20.09.2023
(51) Int. Cl.: G01K 7/42, G01K 13/024, G01L 11/00, G01F 1/684, G01F 1/699, G01F 1/74, G01F 1/34, G01F 15/04, G01F 1/698, G01N 25/18

(54) **SENSORANORDNUNG**
SENSOR ARRANGEMENT
ENSEMBLE CAPTEUR

(30) Priorität: 20.09.2022 DE 102022209905
(43) Veröffentlichungstag der Anmeldung: 30.07.2025
(73) Patentinhaber: Hahn-Schickard-Gesellschaft für angewandte Forschung e.V., 78052 Villingen-Schwenningen (DE)
(72) Erfinder: RAIMANN, Philipp, 78052 Villingen-Schwenningen (DE); BILLAT, Sophie, 78052 Villingen-Schwenningen (DE); HEDRICH, Frank, 78052 Villingen-Schwenningen (DE)
(74) Vertreter: Pfitzner, Hannes
(86) Internationale Anmeldenummer: PCT/EP2023/075915
(87) Internationale Veröffentlichungsnummer: WO 2024/061957

(56) Entgegenhaltungen:
- WO-A1-2022/008212
- DE-A1- 10 309 205
- US-A1- 2020 326 247
- US-A1- 2020 333 273
- FORSYTHE CARLOS ET AL: "3[omega] techniques for measurement of volumetric heat capacity and anisotropic thermal conductivity of a solution processable, hybrid organic/inorganic film, Te-PEDOT:PSS", JOURNAL OF APPLIED PHYSICS, AMERICAN INSTITUTE OF PHYSICS, 2 HUNTINGTON QUADRANGLE, MELVILLE, NY 11747, vol. 131, no. 10, 14 March 2022 (2022-03-14), pages 1 - 9, XP012264183, ISSN: 0021-8979, [retrieved on 20220314], DOI: 10.1063/5.0079328
- LU L ET AL: "3 OMEGA METHOD FOR SPECIFIC HEAT AND THERMAL CONDUCTIVITY MEASUREMENTS", REVIEW OF SCIENTIFIC INSTRUMENTS, AMERICAN INSTITUTE OF PHYSICS, 2 HUNTINGTON QUADRANGLE, MELVILLE, NY 11747, vol. 72, no. 7, 1 July 2001 (2001-07-01), pages 2996 - 3003, XP001102448, ISSN: 0034-6748, DOI: 10.1063/1.1378340

## Beschreibung

Ausführungsbeispiele der vorliegenden Erfindung beziehen sich auf eine Sensoranordnung sowie auf ein entsprechendes Verfahren zur Auswertung. Weitere Ausführungsbeispiele beziehen sich auf einen Flusssensor mit einer entsprechenden Sensoranordnung und/oder einen Drucksensor mit einer entsprechenden Sensoranordnung.

Die Ausgangssignale von thermischen Strömungssensoren (allgemein Flusssensor) werden nicht nur durch die Flussrate (I/min), sondern auch durch die Gaseigenschaften, wie z. B. Dichte ρ, die Wärmeleitfähigkeit k und/oder die spezifische Wärmekapazität c des strömenden Mediums beeinflusst. Die Gaseigenschaften sind wiederum abhängig von der Temperatur und dem Druck. Ändert sich beispielsweise bei einer konstanten Flussrate die Gaszusammensetzung, Temperatur und/oder der Druck, so ändert sich das Ausgangssignal, was fälschlicherweise als Änderung der Flussrate interpretiert werden kann. Daher werden thermische Flusssensoren entweder auf ein Gas/Gasgemisch kalibriert oder die Gaseigenschaften müssen durch weitere zusätzliche Sensoren bestimmt werden, um das resultierende Ausgangssignal mithilfe von Algorithmen zu kompensieren. Die Signalkompensation ist hierbei genauer, wenn sich die Sensoren in unmittelbarer Nähe des Strömungssensors befinden.

Stand der Technik ist es also, Strömungssensoren auf Gaseigenschaften (bekannten Druck, Temperatur und Gaszusammensetzung) zu kalibrieren oder durch zusätzliche eigenständige MEMS-Sensoren (Umweltsensor) eine Signalkompensation zu ermöglichen. Das setzt gemäß dem Stand der Technik die Integration von Sensoren mit unterschiedlichen Messprinzipien voraus. Bei typischen Sensoranordnungen mit geringer Komplexität lässt sich die Wärmeleitfähigkeit sowie die volumetrische Wärmekapazität nicht einfach bestimmen, um so ein Kalibrieren zu ermöglichen. Deshalb besteht der Bedarf nach einem verbesserten Ansatz. Weiterer Stand der Technik ist in der Patentliteratur zu finden. Hierbei sind die Veröffentlichungen: WO 2022/008212 A1, DE 103 09 205 A1, US 2020/333273 A1, US 2020/326247 A1 zu nennen. Weiteren Stand der Technik formen die Veröffentlichungen mit dem Titel "3[omega] techniques for measurement of volumetric heat capacity and anisotropic thermal conductivity of a solution processable, hybrid organic/inorganic film, Te-PEDOT:PSS" und "3 OMEGA METHOD FOR SPECIFIC HEAT AND THERMAL CONDUCTIVITY MEASUREMENTS".

Ausführungsbeispielen der vorliegenden Erfindung liegt die Aufgabe zugrunde, dass ein Konzept geschaffen werden soll, das es ermöglicht, volumetrische Wärmekapazität und Wärmeleitfähigkeit zuverlässig und genau mit einer Messanordnung mit geringer Komplexität zu ermitteln.

Die Aufgabe wird durch den Gegenstand der unabhängigen Patentansprüche gelöst.

Ausführungsbeispiele der vorliegenden Erfindung schaffen eine Sensoranordnung mit mindestens einer Sensorzelle und einer Auswertung. Die mindestens eine Sensorzelle ist thermisch mittels eines Heizers anregbar bzw. zur thermischen Schwingung anregbar. Die Sensorzelle ist ausgebildet, um in Abhängigkeit von einer Gaseigenschaft eines die Sensorzelle umgebenden Gases, insbesondere einer Wärmeleitfähigkeit und/oder volumetrische Wärmekapazität und/oder einer Temperatur und/oder einem Druck ein entsprechendes (thermisches) Schwingungsverhalten auszubilden. Hierbei schwingt entsprechend Ausführungsbeispielen der Heizer, der beispielsweise als freitragende Brückenstruktur ausgelegt ist. Hierbei wird die Sensorzelle mittels einer Anregungsfrequenz angeregt, wobei eine erste Anregungsfrequenz oder eine erste Auswertungsfrequenz für eine erste Messung verwendet wird und wobei eine zweite Anregungsfrequenz oder zweite Auswertefrequenz für eine zweite Messung verwendet wird. Die erste Anregungsfrequenz unterscheidet sich von der zweiten Anregungsfrequenz bzw. die erste Auswertefrequenz unterscheidet sich von der zweiten Auswertefrequenz. Die Auswertung ist ausgebildet, auf Basis der ersten Messung eine Wärmeleitfähigkeit und auf Basis der zweiten Messung eine volumetrische Wärmekapazität zu bestimmen. Die erste Anregungsfrequenz ist um mindestens Faktor 2 oder mindestens Faktor 4 kleiner als die Grenzfrequenz des Sensors, wobei die zweite Anregungsfrequenz um mindestens Faktor 2 oder mindestens Faktor 4 größer ist als die Grenzfrequenz des Sensors; und/oder wobei die erste Auswertefrequenz um mindestens Faktor 2 oder mindestens Faktor 4 kleiner ist als die Grenzfrequenz des Sensors und wobei die zweite Auswertefrequenz um mindestens Faktor 2 oder mindestens Faktor 4 größer ist als die Grenzfrequenz des Sensors.

An dieser Stelle sei angemerkt, dass entsprechend Ausführungsbeispielen die erste Anregungsfrequenz und/oder die zweite Anregungsfrequenz größer gleich 0 Hz betragen kann. Das heißt also, dass für eine erste Messung eine Anregung (oder für eine zweite Messung eine Anregung) mit 0 Hz erfolgen kann, das heißt also eine Anregung mittels eines DC-Stroms beispielsweise. Beispielsweise ist die erste Anregungsfrequenz gleich 0, wobei die Anregungsenergie für die erste Messung größer 0 ist. Bei der zweiten Messung wird dann beispielsweise eine zweite Anregungsfrequenz größer 0 Hz verwendet. Entsprechend Ausführungsbeispielen kann natürlich auch die zweite Anregungsfrequenz gleich 0 Hz sein, nämlich mit einer Anregungsenergie für die zweite Messung größer 0, wobei hier dann die erste Messung mit einer Anregungsfrequenz größer 0 verwendet wird.

Ausführungsbeispielen der vorliegenden Erfindung liegt die Erkenntnis zugrunde, dass bei unterschiedlichen Anregungen, z. B. mit unterschiedlichen Anregungsfrequenzen, differenzierte Sensitivitäten für unterschiedliche physikalische Parameter zugeordnet zu unterschiedlichen physikalischen Gruppen, nämlich für Gruppe 1 (die die Wärmeleitfähigkeit umfasst) und für Gruppe 2 (die die volumetrische Wärmekapazität umfasst), ausgebildet werden können. Aufgrund der unterschiedlichen Anregungen/unterschiedlichen Anregungsfrequenz oder auch aufgrund von unterschiedlichen Auswertefrequenzen werden unterschiedlich hohe Empfindlichkeiten/Sensitivitäten für die unterschiedlichen physikalischen Parameter ausgebildet, was eine unabhängige Bestimmung der volumetrischen Wärmekapazität cv sowie der Wärmeleitfähigkeit k ermöglicht. Hierbei wird also der eine (einzige) Sensor in einem ersten Frequenzbereich betrieben, der eher eine hohe Empfindlichkeit gegenüber der Wärmeleitfähigkeit k ausbildet und sogleich eine geringe Querempfindlichkeit gegenüber der volumetrischen Wärmekapazität cv. Für eine zweite Messung wird (derselbe) Sensor in einem Frequenzbereich betrieben, der eine hohe Empfindlichkeit gegenüber der volumetrischen Wärmekapazität cv ausbildet und sogleich eine geringe Querempfindlichkeit gegenüber der Wärmeleitfähigkeit k besitzt.

Mit anderen Worten ausgedrückt heißt es, dass entsprechend Ausführungsbeispielen zwei Messungen mit unterschiedlichen Anregungen durchgeführt werden. Messung 1 kann mit einer ersten Anregung, die z. B. eine erste Anregungsfrequenz umfasst, ausgeführt werden, während die Messung 2 mit einer unterschiedlichen Anregung, z. B. mit einer größeren oder kleineren, das heißt also unterschiedlichen, Anregungsfrequenz durchgeführt wird. Alternativ wäre es auch denkbar, dass die Anregung 1 eine DC-Anregung (Anregungsfrequenz gleich 0) aufweist, während Messung 2 mit einer unterschiedlichen Anregung mit einer Anregungsfrequenz größer 0 durchgeführt wird. Eine weitere Alternative wäre es, dass der eine Sensor an unterschiedlichen Auswertefrequenzen ausgewertet wird.

Entsprechend Ausführungsbeispielen unterscheidet sich die erste Anregungsfrequenz von der zweiten Anregungsfrequenz um mindestens Faktor 2 oder mindestens Faktor 4 oder sogar mindestens Faktor 10. Analog hierzu kann sich die erste und die zweite Auswertefrequenz um mindestens Faktor 2, mindestens Faktor 4 oder mindestens Faktor 8 unterscheiden. Hierbei können beispielsweise fixe erste Anregungsfrequenzen und zweite Anregungsfrequenzen bzw. fixe Auswertefrequenzen und zweite Auswertefrequenzen verwendet werden.

Hieraus ergibt sich entsprechend Ausführungsbeispielen ein Empfindlichkeitsunterschied bei den zwei Messungen. Beispielsweise kann die Empfindlichkeit bei der zweiten Messung für die volumetrische Wärmekapazität um mindestens Faktor 3 oder mindestens Faktor 4 oder mindestens Faktor 5 höher sein als die Empfindlichkeit bei der ersten Messung für volumetrische Wärmekapazität. Die Empfindlichkeit bei der zweiten Messung für Wärmeleitfähigkeit kann um mindestens Faktor 1,1 oder mindestens Faktor 1,2 höher sein als die Empfindlichkeiten bei der zweiten Messung für Wärmeleitfähigkeit.

Entsprechend Ausführungsbeispielen wird die Sensorzelle mittels einer Anregungsfrequenz bei der ersten und zweiten Messung oder in dem oben beschriebenen Sonderfall bei zumindest einer der zwei Messungen angeregt. Die periodische Anregung kann beispielsweise mittels einer Rechteckspannung erfolgen. Entsprechend weiteren Ausführungsbeispielen wäre es auch denkbar, dass die Anregungsfrequenz variierend ausgelegt ist, beispielsweise als Chirp-Signal oder als Dirac-Signal. Hierbei werden für die Auswertung unterschiedlicher Auswertefrequenzen für die erste und zweite Messung gewählt.

Beide oben erläuterten Varianten haben gemein, dass entsprechend weiteren Ausführungsbeispielen die Messungen zu unterschiedlichen Zeitpunkten erfolgen können (Messung 1 Zeitpunkt t1, Messung 2 Zeitpunkt t2).

Zum Sensor: Entsprechend Ausführungsbeispielen kann die Sensorzelle eine Kavität mit einem Heizer oder eine Wärmesenke mit einem beabstandeten Heizer (oder einen von der Wärmesenke beabstandeten Heizsteg) aufweisen. Der Heizer bzw. Heizsteg kann ausgebildet sein, um thermisch zu schwingen und so das (thermische) Schwingungsverhalten auszubilden. Entsprechend Ausführungsbeispielen wäre es denkbar, dass der Heizer durch den Heizsteg, z. B. in Form einer freitragenden Struktur oder freitragenden Brückenstruktur, gebildet ist.

Entsprechend weiteren Ausführungsbeispielen weist die Sensorzelle einen Detektor auf, der ausgebildet ist, um das Schwingungsverhalten zu detektieren. Der Detektor kann separat zum Heizer angeordnet sein. Entsprechend weiteren Ausführungsbeispielen kann der Detektor auch in den Heizer wie folgt integriert sein. Der Heizer wird zur (thermischen) Schwingung angeregt, wobei dann eine resistive Auswertung des Temperatursignals im selben Heizer erfolgen kann. Der Heizer ist entsprechend Ausführungsbeispielen stromdurchflossen, z. B. aus Metall oder einem anderen leitfähigen Material, ausgelegt.

Entsprechend Ausführungsbeispielen ist die Auswertung ausgebildet, das Schwingungsverhalten des Sensors anhand der dynamischen Temperaturantwort und/oder anhand der Amplitude und/oder anhand der Frequenz und/oder anhand der Phase zu bestimmen. Entsprechend Ausführungsbeispielen kann die Auswertung als ASIC implementiert sein. Hierbei kann der ASIC in einen Chip oder monolithisch in dem Chip, der auch die Sensorzelle beherbergt, integriert sein.

Weitere Ausführungsbeispiele schaffen einen Flusssensor mit einer entsprechenden Sensoranordnung. Der Flusssensor ist ausgebildet, einen Fluss (Volumenfluss oder Gasfluss) unter Berücksichtigung der bestimmten Wärmeleitfähigkeit und volumetrischen Wärmekapazität zu bestimmen. Hierbei ist vorteilhaft, dass die Bestimmung in kompensierter Weise erfolgt.

Weitere Ausführungsbeispiele schaffen einen Drucksensor, der ausgebildet ist, um den Druck unter Berücksichtigung der volumetrischen Wärmekapazität und/oder Wärmeleitfähigkeit zu bestimmen.

Bei beiden gerade erläuterten Anwendungen des Drucksensors und des Flusssensor ist es vorteilhaft, dass durch die Bestimmung von Wärmeleitfähigkeit und Wärmekapazität das Gas bzw. Gasgemisch unbekannt sein kann, so dass dennoch der korrekte Volumenfluss oder der korrekte Druck bestimmt wird.

Ein weiteres Ausführungsbeispiel schafft ein Verfahren mit folgenden Schritten:
- Anregen des Sensors (10) mittels einer Anregungsfrequenz, wobei eine erste Anregungsfrequenz oder eine erste Auswertungsfrequenz für eine erste Messung (M1) verwendet wird, und wobei eine zweite Anregungsfrequenz oder Auswertefrequenz für eine zweite Messung (M2) verwendet wird, wobei sich die erste Anregungsfrequenz von der zweiten Anregungsfrequenz unterscheidet oder wobei sich die erste Auswertefrequenz von der zweiten Auswertefrequenz unterscheidet; und
- Bestimmen auf Basis der ersten Messung (M1) eine Wärmeleitfähigkeit (k) und auf Basis der zweiten Messung (M2) eine volumetrische Wärmekapazität (cv),
wobei die erste Anregungsfrequenz um mindestens Faktor 2 oder mindestens Faktor 4 kleiner ist als die Grenzfrequenz des Sensors und wobei die zweite Anregungsfrequenz um mindestens Faktor 2 oder mindestens Faktor 4 größer ist als die Grenzfrequenz des Sensors; und/oder wobei die erste Auswertefrequenz um mindestens Faktor 2 oder mindestens Faktor 4 kleiner ist als die Grenzfrequenz des Sensors und wobei die zweite Auswertefrequenz um mindestens Faktor 2 oder mindestens Faktor 4 größer ist als die Grenzfrequenz des Sensors.

Entsprechend weiteren Ausführungsbeispielen kann das Verfahren computerimplementiert sein.

Bevor nachfolgend Ausführungsbeispiele der vorliegenden Erfindung anhand der beiliegenden Zeichnungen erläutert werden, sei darauf hingewiesen, dass gleichwirkende Elemente und Strukturen mit den gleichen Bezugszeichen versehen sind, so dass die Beschreibung derer aufeinander anwendbar bzw. austauschbar ist.
- Fig. 1a und 1b: zeigt in einer Schnittdarstellung (Fig. 1a) einer Draufsicht (Fig. 1b) schematisch eine Sensorzelle zur Verwendung in Ausführungsbeispielen;
- Fig. 2a-2q: zeigen schematische Darstellungen von Sensorzellen zur Verwendung in erweiterten Ausführungsbeispielen;
- Fig. 3a und 3b: zeigen ein schematisches Ausführungsbeispiel einer Sensoranordnung mit zwei Sensorzellen gemäß einem Vergleichsaspekt;
- Fig. 3c und 3d: zeigen schematische Diagramme zur Illustration möglicher Sensorzellendimensionen gemäß Ausführungsbeispielen;
- Fig. 4a und 4b: zeigen schematische Diagramme zur Erläuterung der Empfindlichkeit gegenüber Wärmeleitfähigkeit und volumetrische Wärmekapazität für zwei Sensorzellen gemäß Vergleichsaspekten;
- Fig. 5: zeigt ein schematisches Blockschaltbild einer Sensorzelle mit einer Auswerteelektronik gemäß einem Hauptausführungsbeispiel;
- Fig. 6a und 6b: zeigen schematische Diagramme zur Erläuterung der Empfindlichkeit gegenüber Wärmeleitfähigkeit und volumetrische Wärmekapazität für zwei Sensorzellen gemäß Ausführungsbeispielen;
- Fig. 7a und 7b: zeigen schematische Diagramme zur Illustration der Empfindlichkeiten aufgetragen über die Frequenz zur Erläuterung möglicher Auslegungen gemäß Ausführungsbeispielen;
- Fig. 8: zeigt ein schematisches Diagramm zur Illustration der Abhängigkeit zwischen Sensorempfindlichkeit und Messgas;
- Fig. 9a, 9b, 9c und 9d: zeigen schematische Darstellungen von möglichen Anwendungen gemäß Ausführungsbeispielen; und
- Fig. 10a-10c: schematische Darstellungen zur Erläuterung einer Auswertung gemäß einem erweiterten Ausführungsbeispiel.

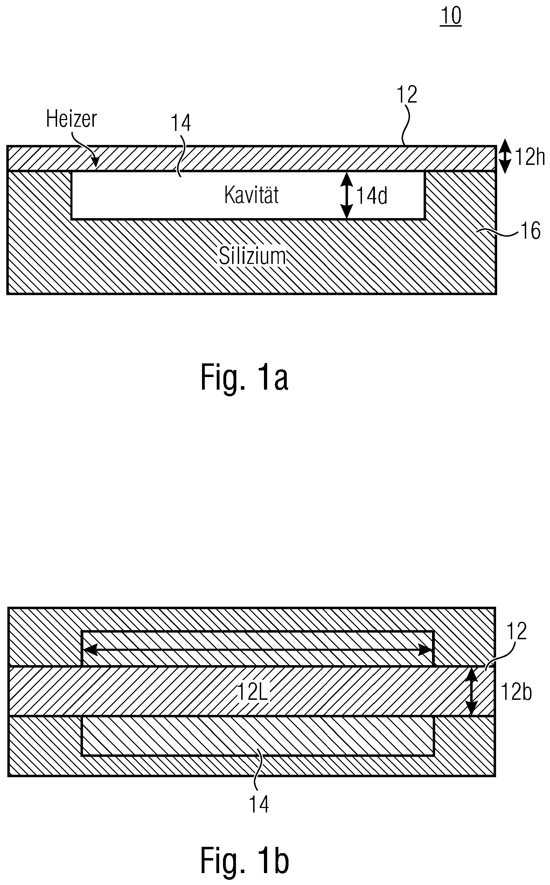

Fig. 1 zeigt in Abbildung a eine Schnittdarstellung einer Sensorzelle 10 und in der Fig. 1b eine Draufsicht. Die Sensorzelle 10 umfasst einen Heizer 12, der beispielsweise als freitragende Struktur über einer Kavität 14 angeordnet ist. Die Kavität 14 kann beispielsweise in ein Siliziumsubstrat 16 eingebettet sein. Der Heizer 12 kann durch Anregung mittels einer Ansteuerfrequenz zur thermischen Schwingung angeregt werden. Hierbei wird derselbe beispielsweise mittels einer Wechselspannung/-strom mit der entsprechenden Frequenz durchflossen. Ausgehend von der Anregung stellt sich eine frequenzabhängige Temperaturüberhöhe sowie ggf. ein Tiefpassverhalten ein. Diese Temperaturüberhöhe bzw. dieses Tiefpassverhalten ist von Geometrieparametern und Materialeigenschaften abhängig. Wesentliche Geometrieparameter sind beispielsweise die Höhe des Heizers 12h, die Breite des Heizers 12b sowie die Länge des Heizers 12l. Ein weiterer Parameter ist das Volumen der Kavität 14, das im Wesentlichen von der Höhe der Kavität 14d abhängig ist. Das Siliziumsubstrat 16 bzw. die Unterseite der Kavität 14 dient als Temperatursenke für den Heizer 12, wobei die Wärmeübertragung in die Temperatursenke von der Dicke 14d der Kavität 14 abhängt.

Eine so zur Schwingung angeregte Sensorzelle 10 ist ausgebildet, um mit einer entsprechenden Frequenz zu schwingen. Diese Frequenz ist abhängig von physikalischen Parametern des umgebenden Gases, sowohl aufseiten der Kavität 14 als auch auf der der Kavität 14 gegenüberliegenden Seite, die beispielsweise als Messseite dient. Einflussfaktoren sind beispielsweise die Temperatur, der Druck, insbesondere aber die Wärmeleitfähigkeit und die volumetrische Wärmekapazität. Das ermöglicht umgekehrt, dass ausgehend von dem Schwingungsverhalten des Heizers diese physikalischen Parameter Wärmeleitfähigkeit und/oder volumetrische Wärmekapazität bestimmt werden können. Hierzu wird das Schwingungsverhalten beispielsweise mittels eines Detektor (nicht dargestellt) überwacht.

Das Verfahren lässt sich wie folgt bestimmen:
- Heizer wird periodisch angeregt (Strom oder Spannung) und erwärmt sich (Joulesche Wärme).
- Temperatur des Heizers variiert und ist abhängig vom Wärmetausch mit dem umgebenden Gas (das die freitragende Heizstruktur 12 umgebende/zu analysierende Gas).
- Wärmeleitfähigkeit und volumetrische Wärmekapazität beeinflussen dynamische Wärmeabgabe an das Gas.
- als Folge dessen kann die dynamische Temperaturantwort des Heizers 12 (z. B. Amplitude und Phase) gemessen werden und zur Detektion kann eine resisitive oder thermoelektrische Überwachung der thermischen Antwort erfolgen. Hierdurch stellt sich beispielsweise folgende Heizübertragungstemperatur T ein. T = Funktion (L, h, b, d, kₕ, cvₕ, k_{gas}, cv_{gas}) mit folgenden Ersatzparametern: R_{Heizer} = L / (h*b*kₕ); C_{Heizer} = cvₕ*b*h*L; R_{Gas}=d / (L*b*k_{gas}); C_{Gas}=d * b*L*cv_{gas}.

Wenn Gase unterschiedliche k_{gas} und cv_{gas} aufweisen, ist die Amplitude und das dynamische Verhalten mit der Frequenz unterschiedlich. Das System zeigt ein Hochpassverhalten, d. h. die Temperatur nimmt mit steigender Frequenz ab und die Phasenverschiebung nimmt mit steigender Frequenz zu. Dieses frequenzabhängige Verhalten kann neben der Sensordimensionierung auch von den Gaseigenschaften abhängen. Wird das Gleichgewicht zwischen den Skalierungsfaktoren für Breite vom Heizer b und die Breite der effektiven Wärmeübertragungsfläche an das Gas b_{gas} angenommen, wird die Grenzfrequenz durch folgenden Zusammenhang beschrieben. Für b_{gas} = b lassen sich die Skalierungsfaktoren eliminieren und es gilt: ${f}_{Grenz} = \frac{h ⋅ {k}_{h}}{2 ⋅ π ⋅ {L}^{2} \left({cv}_{h}⋅h+{cv}_{gas}⋅d\right)} + \frac{{k}_{gas}}{2 ⋅ π ⋅ d \left({cv}_{h}⋅h+{cv}_{gas}⋅d\right)}$

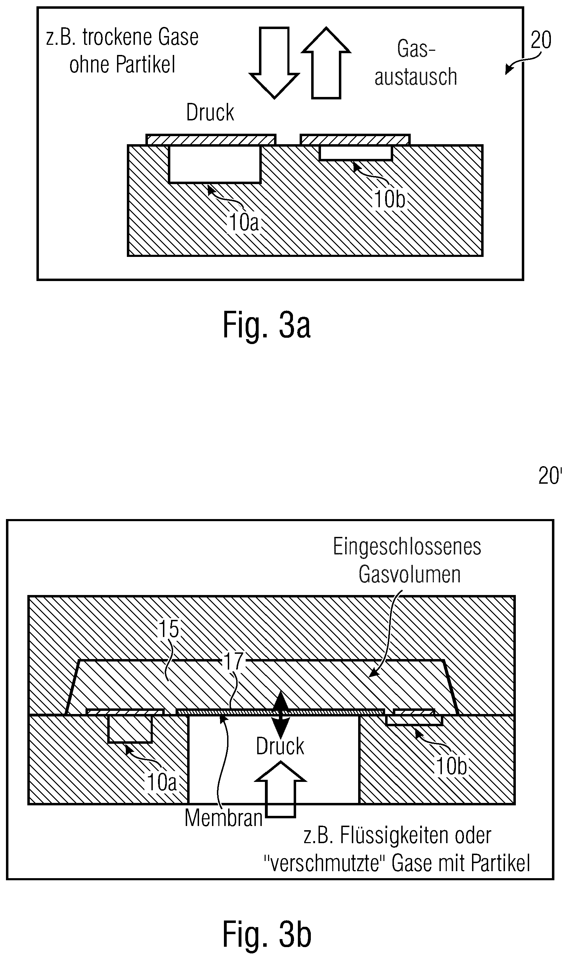

Hierdurch lässt sich ableiten, dass die Grenzfrequenz umso kleiner wird, je geringer die Wärmeleitfähigkeit und höher die volumetrische Wärmekapazität ist. Das heißt also, dass eine Druckerhöhung die Grenzfrequenz des Systems verringert. Eine Temperaturerhöhung vergrößert die Grenzfrequenz des Systems. Dieser Hintergrund aus physikalischer Sicht führt zur Erkenntnis der Erfindung, dass durch die Verwendung von zwei Sensorzellen unterschiedlicher Dimensionierung (ggf. mit unterschiedlicher Ansteuerfrequenz), wie sie beispielsweise in Fig. 3a oder 3b gezeigt sind, erreicht werden kann, dass die Wärmeleitfähigkeit k_{gas} bzw. Sₖ sowie die volumetrische Wärmekapazität S_{cv} bzw. cv_{gas} unabhängig voneinander bestimmt werden kann. Hierbei wird beispielsweise ein Sensor mit einer hohen Empfindlichkeit gegenüber der Wärmeleitfähigkeit in Kombination mit einem Sensor mit einer hohen Empfindlichkeit gegenüber der volumetrischen Wärmekapazität eingesetzt.

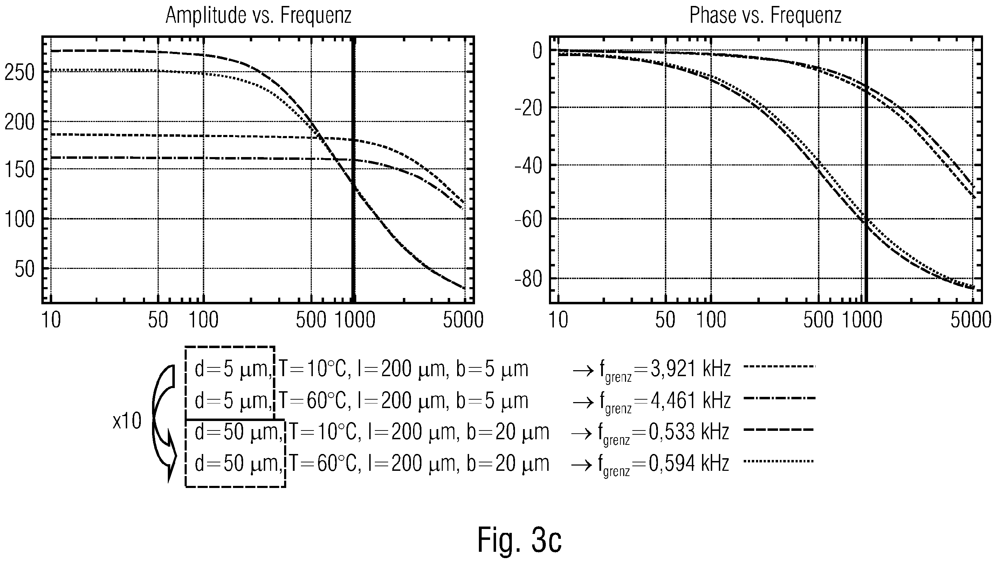

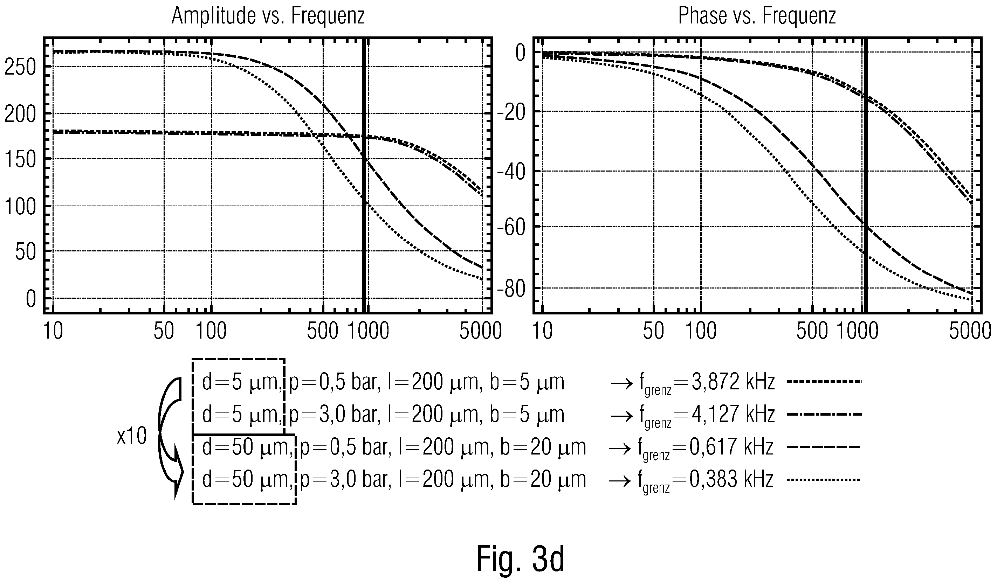

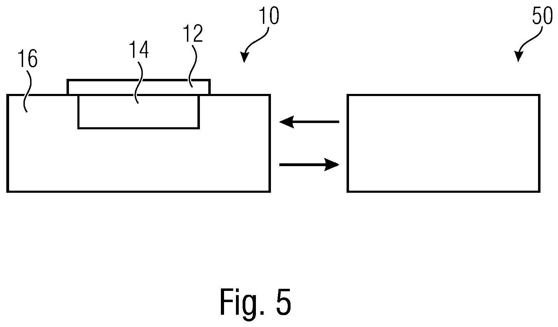

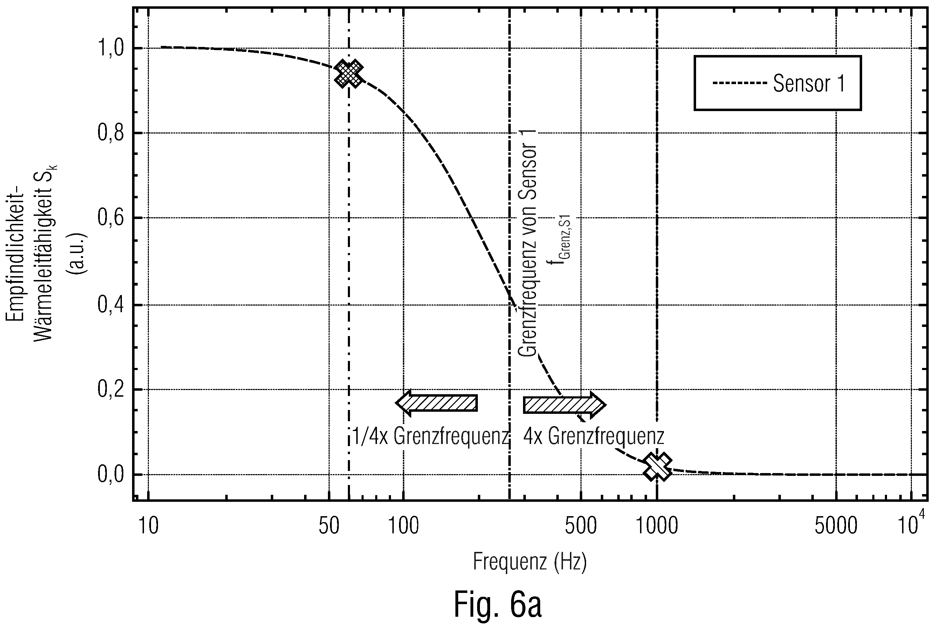

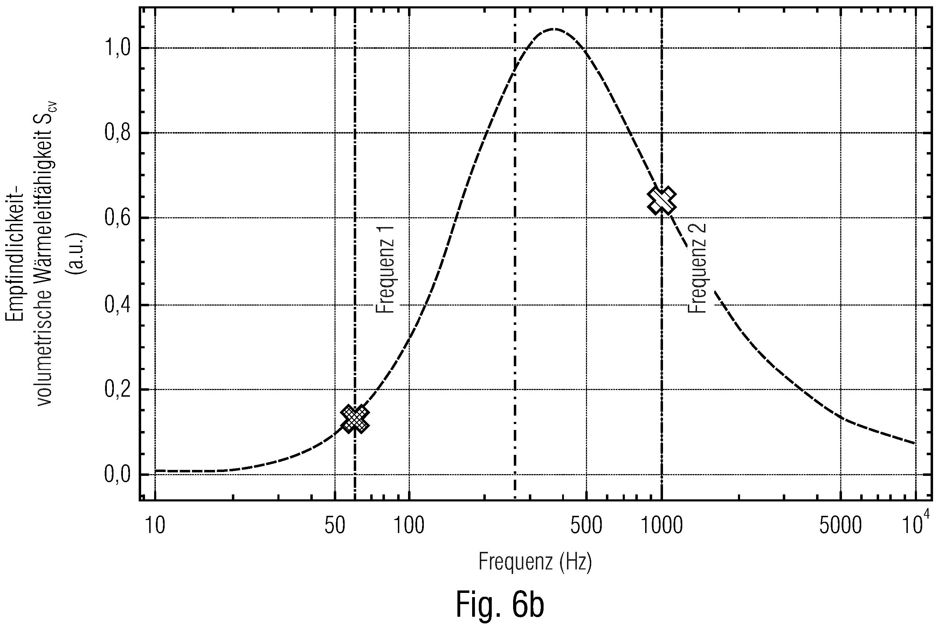

Entsprechend Ausführungsbeispielen besteht der Sensor aus mindestens einem freistehenden Heizelement mit umgebendem Gasvolumen, das periodisch geheizt und dessen Temperatur-Antwort bestimmt wird. Entsprechend Ausführungsbeispielen werden Sensoren entweder gleich oder unabhängig voneinander mittels temperaturabhängigen Widerständen und/oder Thermoelementen ausgelesen:
▪ Variante 1, siehe Fig. 5 und 6: Mindestens ein Sensor wird bei zwei festen Frequenzen oder über zwei Frequenzbereichen gescannt, wo der thermische Sensor einen ausreichend hohen Unterschied bei der Empfindlichkeit gegenüber Wärmeleitfähigkeit und volumetrische Wärmekapazität aufweist
▪ Variante 2, siehe Fig. 3: Zwei oder mehrere Sensoren (Sensorarrays) werden durch geometrische Parametervariation (Länge, Breite, Schichtdicke, Form, Höhe der Kavität) und/oder unterschiedlicher Materialeigenschaften so ausgelegt, dass sich die thermische Anbindung an die Wärmesenke unterscheidet und kombiniert, so dass sie in der Empfindlichkeit gegenüber Wärmeleitfähigkeit und volumetrische Wärmekapazität beim Betrieb in einem oder mehreren gewählten Frequenzbereich(en) oder fixen Frequenzen ausreichend variiert.

Fig. 3a zeigt als Vergleichsbeispiel eine Sensoranordnung 20 mit einem ersten Sensor 10a und einem zweiten Sensor 10b. Wie zu erkennen ist, sind die Sensoren unterschiedlich groß dimensioniert, wobei das Grundprinzip dem aus Fig. 1a und Fig. 1b entspricht. Beide Sensorzellen 10a und 10b sind mit ihrer der Kavität abgewandten Seite einem zu untersuchenden Gas zugewandt bzw. so in eine Vorrichtung eingebettet, dass hier ein Gasaustausch, z. B. mit trockenen Gasen ohne Partikel, erfolgen kann.

Mögliche Variationsparameter für die unterschiedlichen Dimensionierung der (zwei) Sensorzellen sind beispielsweise :
- Geometrische Parameter (Länge, Breite, Schichtdicke)
- Ein Heizer kann aus mehreren Heizern aufgebaut sein (z.B. zwei Heizer parallel, Kombination mehrerer Heizer-Formen)
- Heizer-Formen (Löcher z. B. Wabenstrukturen, mit Membran, Mäander, ....)
- Materialien/-kombinationen (thermische Eigenschaften, Passivierung, ...)

Fig. 3b zeigt einen etwas variierten Aufbau, bei welchem die Sensoren 10a und 10b in der Sensoranordnung 20' über ein eingeschlossenes Volumen indirekt angekoppelt sind. Das eingeschlossene Volumen ist mit dem Bezugszeichen 15 versehen und gegenüber der Umgebung über eine Membran 17 gekapselt. Auf diese Membran 17 wirkt das zu messende Gas bzw. das zu messende Medium Flüssigkeit oder verschmutztes Gas mit Partikel ein.

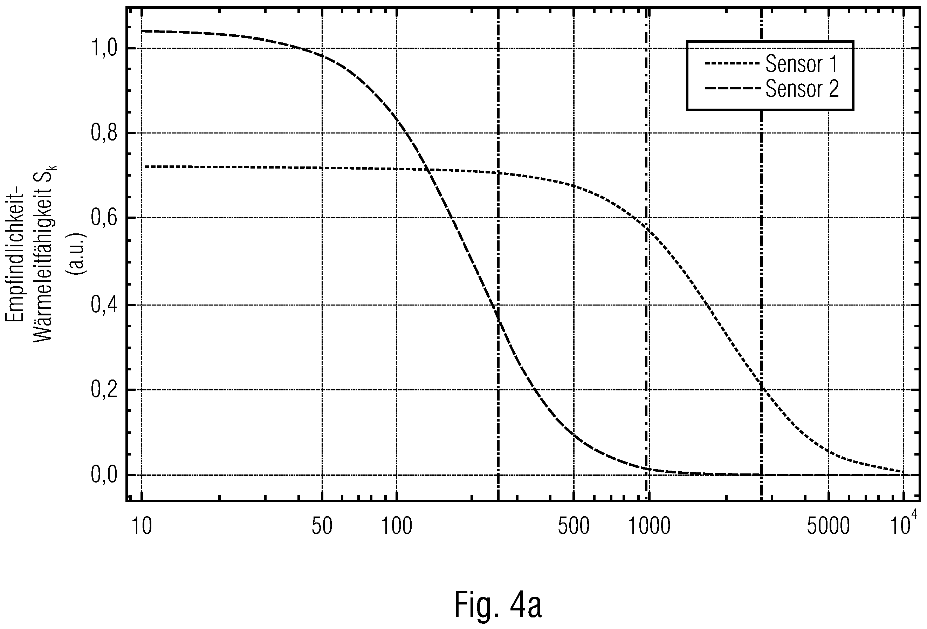

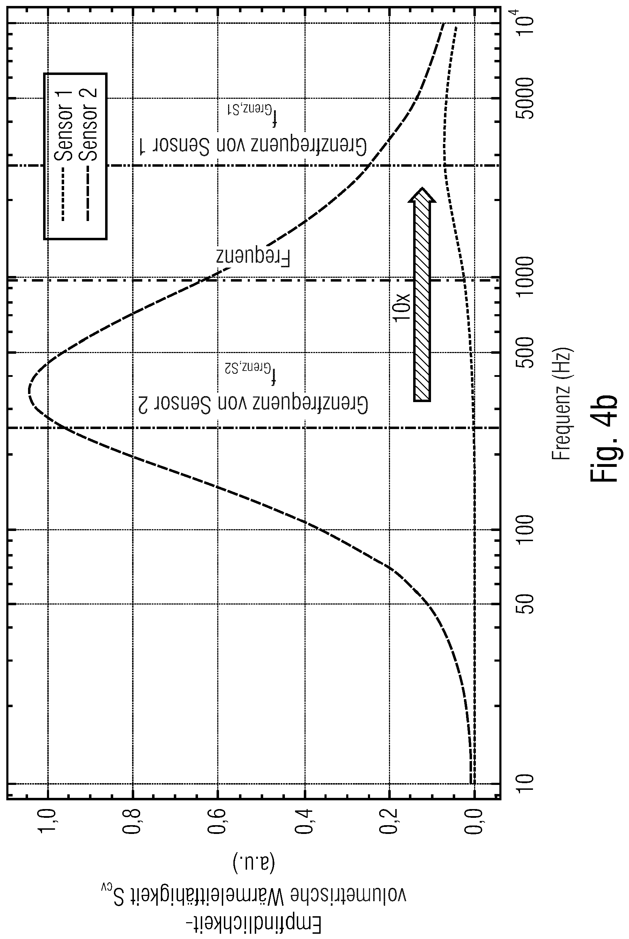

Wie deutlich zu erkennen ist, ist sowohl bei der Ausführung aus Fig. 3a als auch aus der Ausführung aus Fig. 3b die Dimensionierung der Sensorzellen 10a und 10b unterschiedlich. Insofern stellen sich unterschiedliche Sensitivitäten gegenüber den zu messenden Größen volumetrischer Wärmekapazität cv und Wärmeleitfähigkeit k ein. Dieses Prinzip wird beispielsweise anhand der Fig. 4a und 4b deutlich.

Fig. 4a zeigt die Empfindlichkeit für die Wärmeleitfähigkeit Sₖ aufgetragen über die Frequenz für zwei verschiedene Sensoren, während Fig. 4b die Empfindlichkeit für die volumetrische Wärmekapazität S_{cv}, wiederum aufgetragen über die Frequenz, für dieselben zwei Sensoren zeigt. Es ist zu erkennen, dass Sensor 2, insbesondere in einem Frequenzbereich von 10 - 100 Hz, zwar empfindlicher ist als der Sensor 1, wobei der Sensor 1 über dem gesamten Frequenzbereich oder zumindest bis 1000 Hz eine bessere Empfindlichkeit ausbildet. Der Unterschied zwischen den Empfindlichkeiten zwischen Sensor 1 und Sensor 2 ist nicht signifikant in diesen Beispielen, so dass es hier auf den Betriebspunkt von Sensor 1 und Sensor 2 ankommt. Wenn beispielsweise der Betriebspunkt bei 350 Hz gewählt wird, ist Sensor 1 für die Empfindlichkeit der Wärmeleitfähigkeit zu bevorzugen. An dieser Stelle sei angemerkt, dass die Diskussion der Betriebspunkte nur exemplarisch ist und von Sensor zu Sensor variiert. Im Diagramm aus Fig. 4b ist die Empfindlichkeit für die volumetrische Wärmekapazität S_{cv} für die zwei Sensoren 1 und 2 aufgetragen. Wie zu erkennen ist, bildet sich hier eine signifikant höhere Empfindlichkeit bei Sensor 2 im Vergleich zu Sensor 1 aus. Hierbei würde man wohl den Betriebspunkt im Bereich zwischen 350 und 1000 Hz wählen und Sensor 2 bei der Grenzfrequenz f_{Grenz,S2} betreiben. In diesem Bereich bildet vorteilhafterweise Sensor 1 eine gute Empfindlichkeit für Wärmeleitfähigkeit aus, so dass bei Betrieb der zwei Sensoren mit gleicher Anregungs- bzw. Auswertefrequenz die volumetrische Wärmekapazität und die Wärmeleitfähigkeit unabhängig voneinander mit den zwei verschiedenen Sensoren bestimmt werden kann.

Entsprechend einem weiteren Vergleichsbeispiel kann der Betriebspunkt je Sensor variieren. Bevorzugterweise würde man Sensor 2 für die Bestimmung der Wärmeleitfähigkeit unterhalb, z. B. bei ⅓ seiner Grenzfrequenz f_{Grenz,S2}, betreiben, um die Wärmeleitfähigkeit zu bestimmen. Sensor 2 würde man im Bereich der Grenzfrequenz oder etwas höher als die Grenzfrequenz f_{Grenz,S2} betreiben, um die volumetrische Wärmekapazität zu bestimmen.

Hieraus ergeben sich unterschiedliche Betriebsmodi entsprechend unterschiedlichen Vergleichsbeispielen:
- Unterschiedliche Dimensionierung + unterschiedliche Frequenzen
- Unterschiedliche Dimensionierung + gleiche Frequenzen

Zur Auswertung: Wenn bei Ausführungsbeispielen immer von Frequenz gesprochen wird, kann entweder von Anregungsfrequenz oder auch von Auswertefrequenz ausgegangen werden. Beispielsweise kann der Sensor bei einer bestimmten Frequenz angeregt werden und bei einer weiteren ausgewertet werden. Das ist beispielsweise dann vorteilhaft, wenn ein Chirp-Signal oder ein Dirac-Signal verwendet wird und hier sozusagen unterschiedliche Frequenzen durchgespielt werden. Alternativ können auch fixe Anregungsfrequenzen für beide Sensoren oder für die jeweiligen Sensoren 10a und 10b oder für 2 Messungen verwendet werden.

Obige Vergleichsbeispiele haben also gemein, dass zwei thermische Sensoren bzw. mindestens zwei thermische Sensoren unabhängig voneinander angeregt bzw. ausgelesen werden können, so dass die unterschiedlichen Empfindlichkeiten der zwei Sensoren, die entweder unterschiedlich betrieben oder unterschiedlich ausgelegt sind, ausgenutzt werden. Die Empfindlichkeiten lassen sich wie folgt berechnen: ${S}_{k} = \frac{\partial T \left({k}_{gas}, {cv}_{gas}, b, L, h, d, {k}_{h}, {cv}_{h}\right)}{\partial {k}_{gas}}$ ${S}_{cv} = \frac{\partial T \left({k}_{gas}, {cv}_{gas}, b, L, h, d, {k}_{h}, {cv}_{h}\right)}{\partial {c}_{vgas}} .$

Wie oben bereits erläutert, können über den Betriebspunkt die Empfindlichkeiten eingestellt werden.

Alle oben genannten Vergleichsbeispiele haben also gemein, dass mindestens zwei Sensoren/Sensorzellen mit unterschiedlichen Dimensionierungen, z. B. mindestens eine Größenordnung unterschiedlich, kombiniert sind. Diese können beispielsweise auf einem Siliziumchip integriert sein (d. h. monolithisch) und so verschiedene dynamische Verhalten bei Wärmeausbreitung in Gasen ausbilden. Unterschiedliche Betriebspunkte schaffen analog zu unterschiedlichen Dimensionierungen der Sensoren die Basis, dass durch Bestimmung der Amplitude und/oder der Phasenlage des Heizers bei dynamischer Anregung eine Bestimmung der Gaseigenschaften ermöglicht wird.

Typische Dimensionen von Sensorzellen werden nachfolgend für ein exemplarisches Beispiel angegeben. Hier können alle Dimensionen in Kombination oder auch nur für sich alleine Verwendung finden:
▪ Länge des Heizers: 10 - 1000 µm
▪ Breite des Heizers: 1 - 200 µm
▪ Breite der Wärmeübertragung: 1 - 500 µm
▪ Höhe des Heizers: 0,1 - 2 µm
▪ Höhe der Kavität: 0,05 - 500 µm

Mit diesen Dimensionen sind Sensorzellen unterschiedlicher Dimensionierung und damit unterschiedlichen Schwingungsverhalten herstellbar. Das Schwingungsverhalten äußert sich insbesondere anhand der Grenzfrequenz f_{Grenz}. In Fig. 3c sind vier verschiedene Sensoren mit unterschiedlichen Grenzfrequenzen und damit mit unterschiedlichen Schwingungsverhalten aufgelistet bzw. in entsprechenden Diagrammen (Amplitude vs. Phase sowie Phase vs. Frequenz) illustriert. Wie zu erkennen ist, kann durch die Variation der Kavitätshöhe d eine signifikante Verschiebung der Grenzfrequenz um beispielsweise Faktor 10 erreicht werden. Dieser Sensor ist für einen konstanten Druck bei beispielsweise 1 bar und schwankende Temperaturen im Bereich von 10 - 60°C ausgelegt. Auch bei konstanter Temperatur (z. B. 24°C) und schwankendem Druck im Bereich von 0,5 - 3,0 bar zeigt sich ein ähnliches Bild, wie anhand von Fig. 3d zu erkennen ist. Hier sind ebenfalls vier Sensoren dargestellt, wobei wiederum zu erkennen ist, dass die Kavitätshöhe (allgemein Höhe des Heizstabes über der Wärmesenke) einen signifikanten Einfluss auf die Grenzfrequenz f_{Grenz} hat.

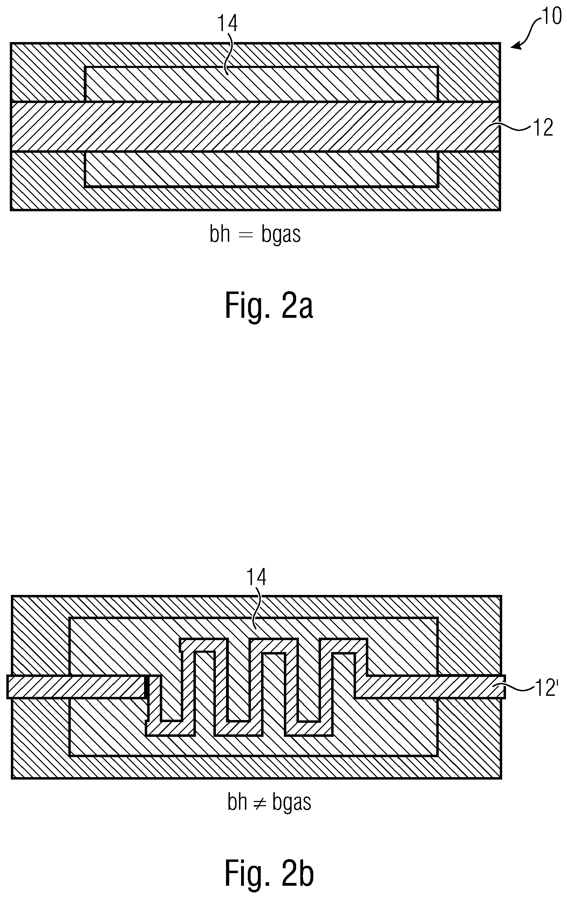

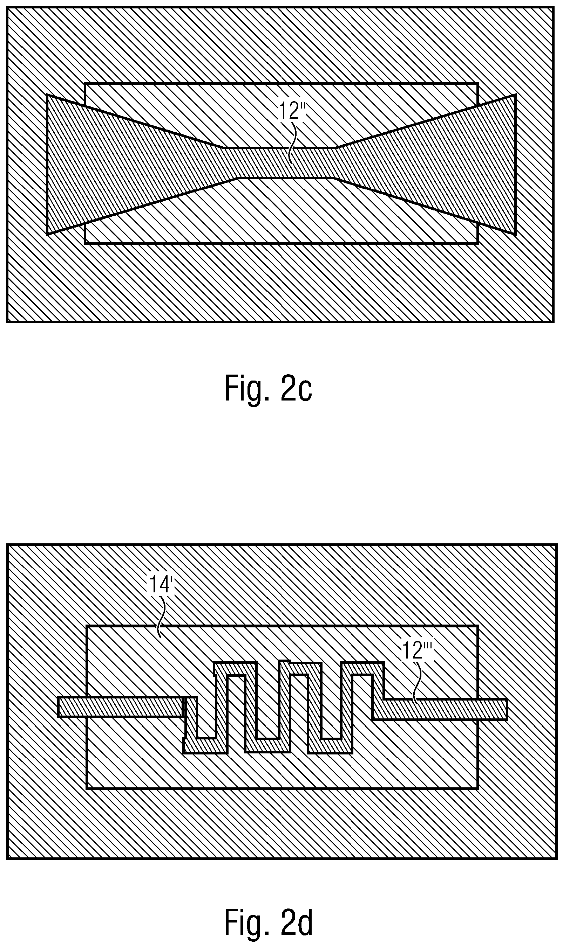

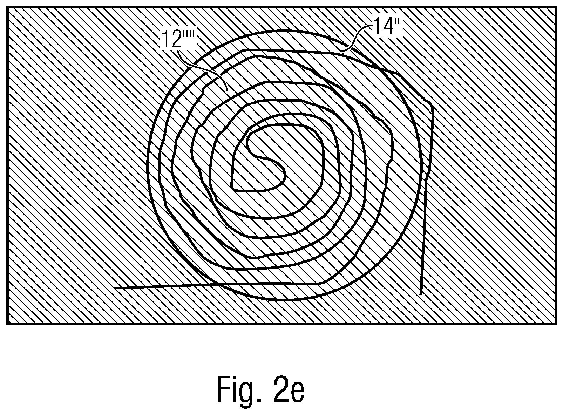

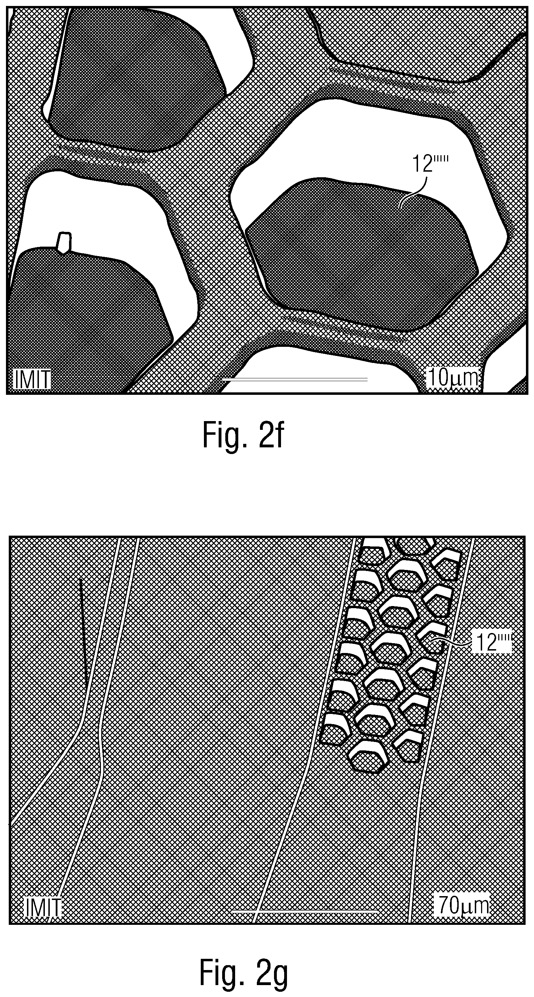

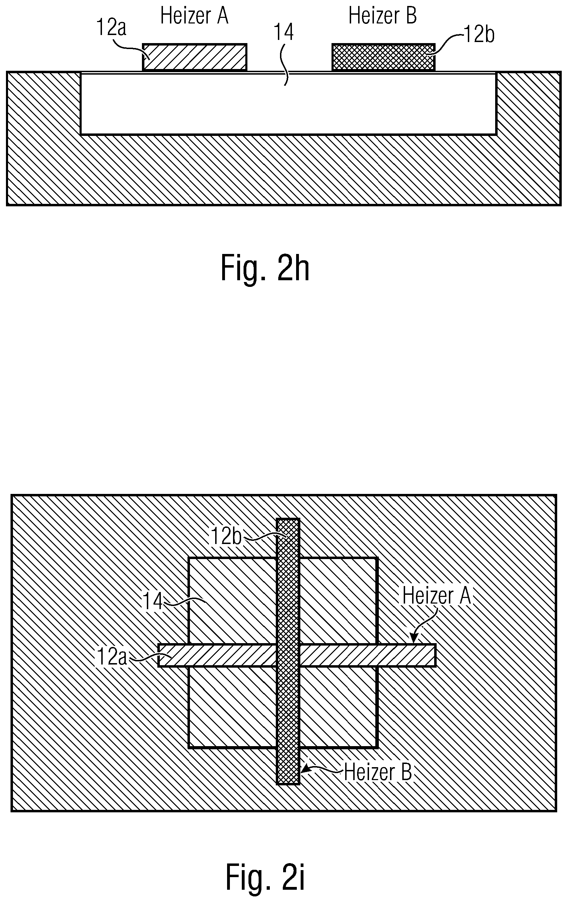

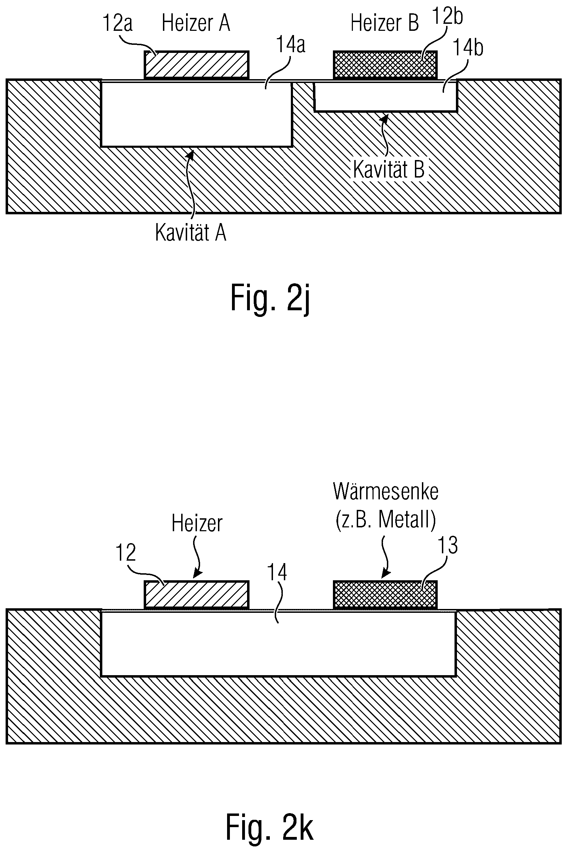

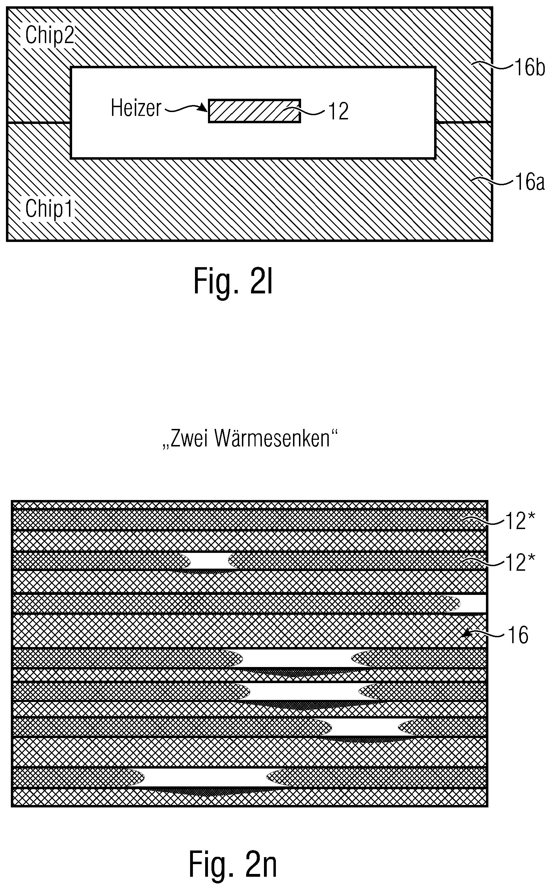

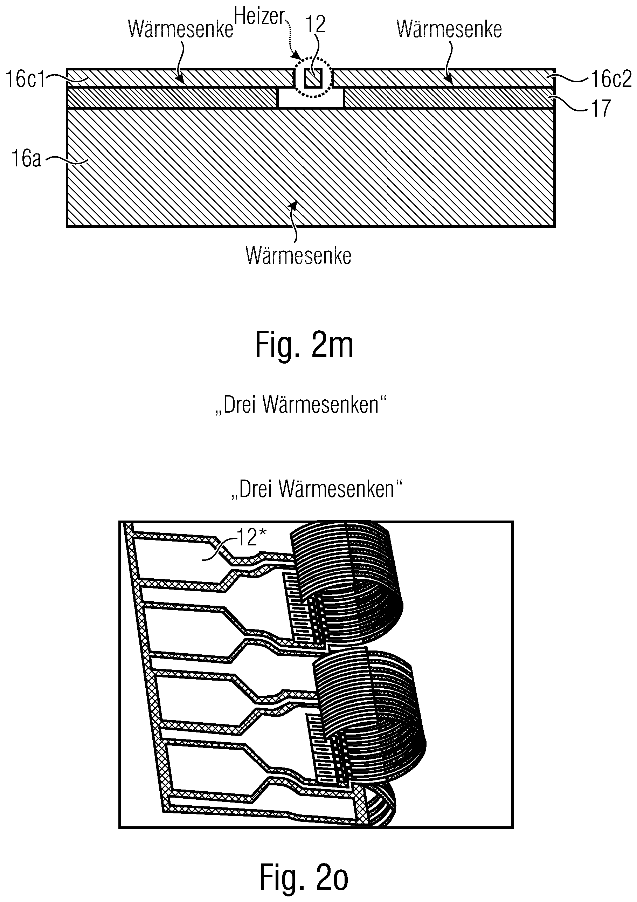

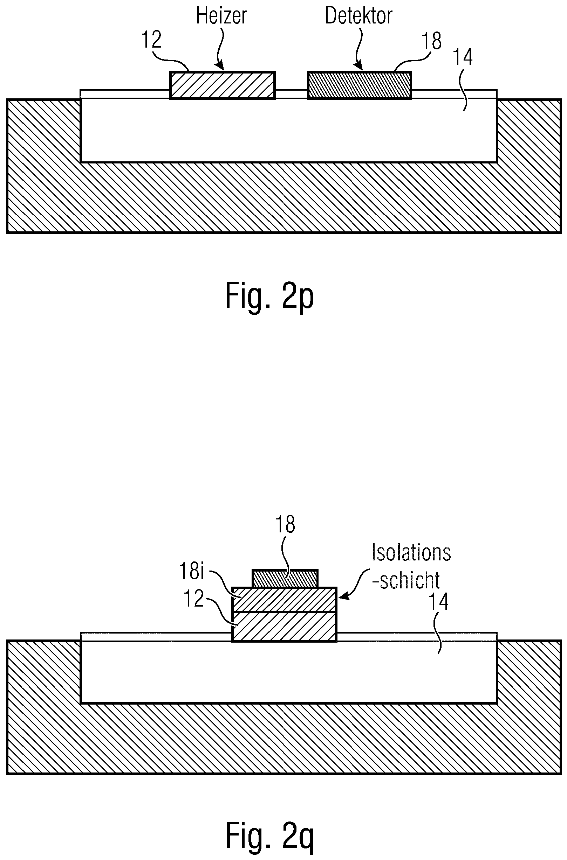

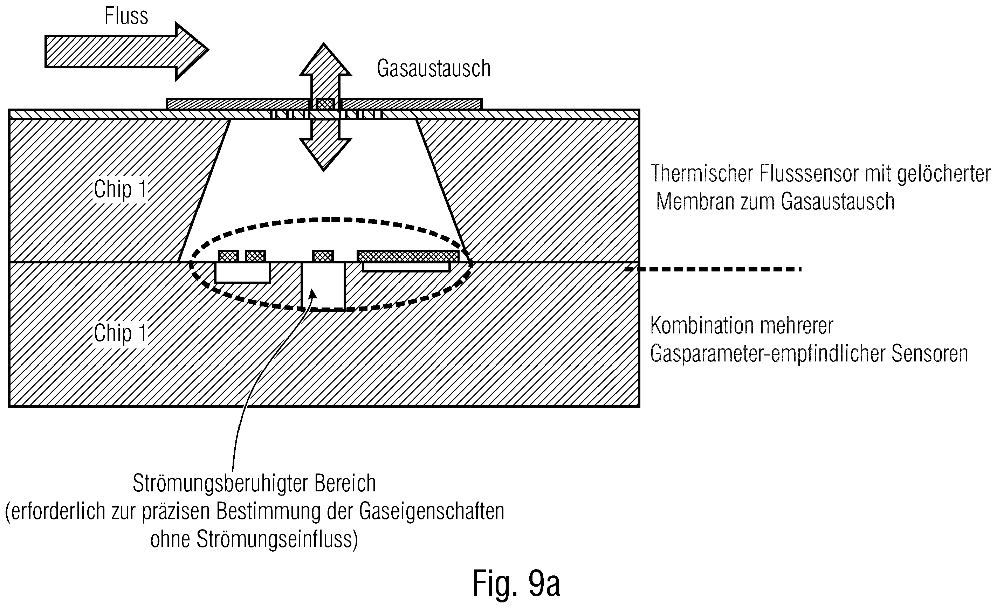

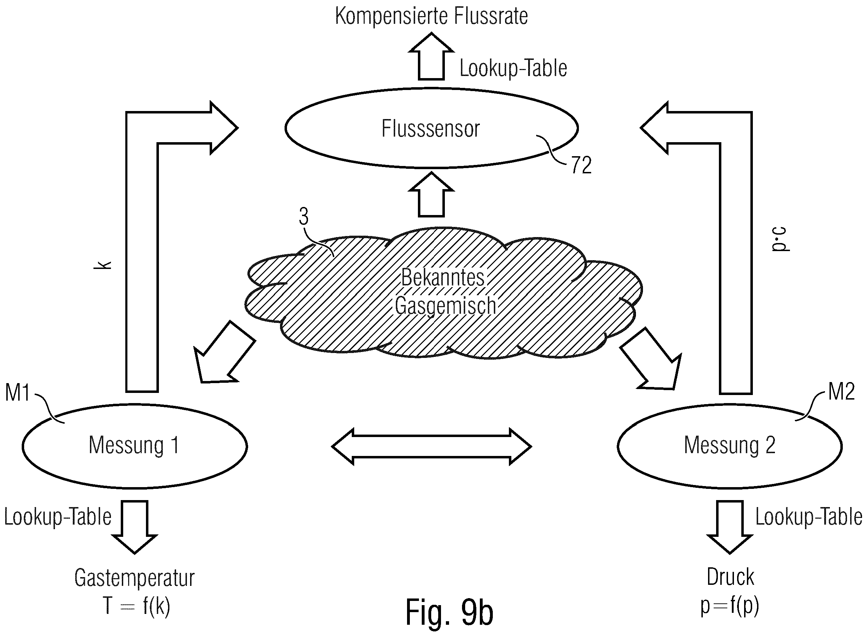

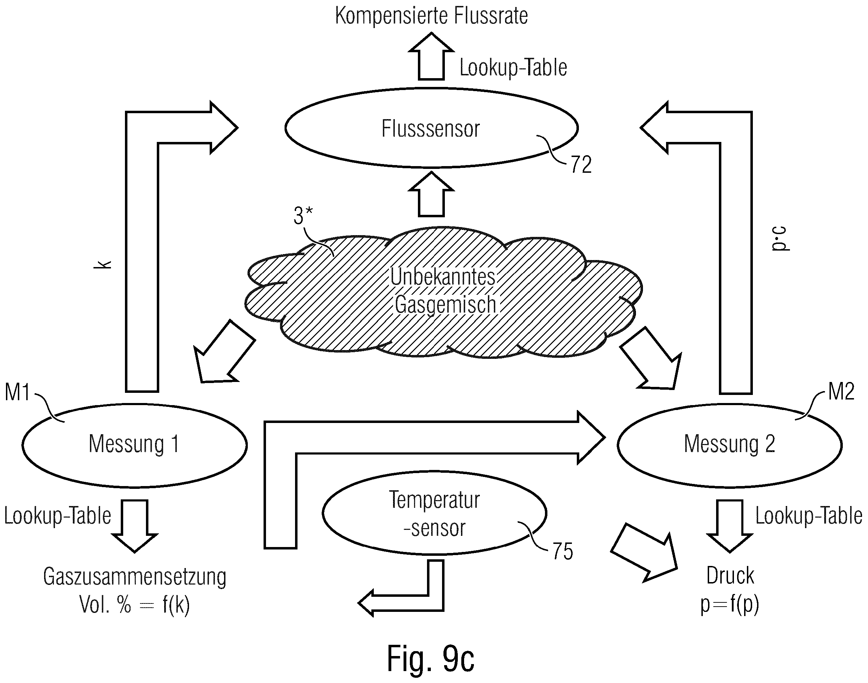

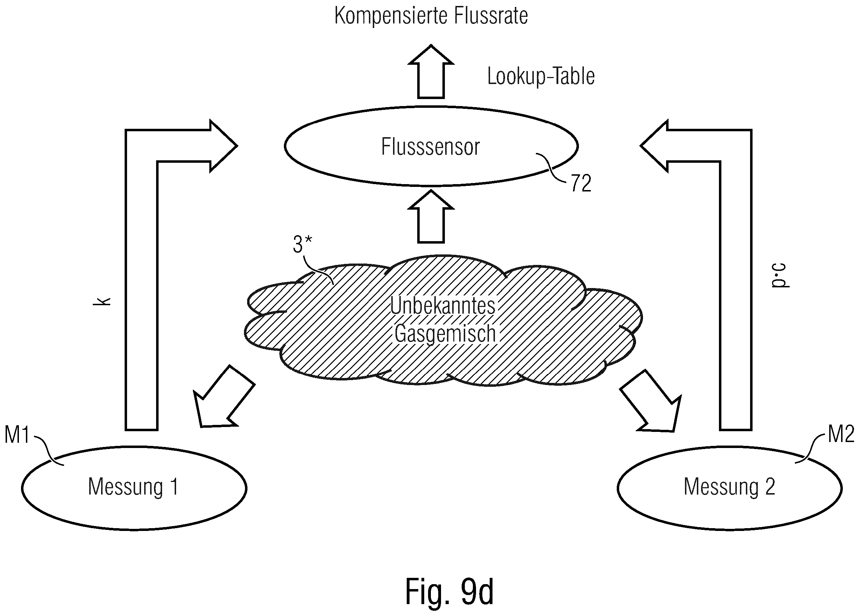

Zur Anregung: Bei obigen Vergleichsbeispielen wurde z. B. davon ausgegangen, dass der Heizer periodisch mittels eines Rechtecksignals und eines Sinussignals angeregt wird, wobei das Ansprechverhalten des Heizers, d. h. also das Schwingungsverhalten bzw. das thermische Schwingungsverhalten des Heizers, durch einige wenige Thermoelemente oder Widerstandsänderungen überwacht werden kann. Das Ergebnis der Modellierung, bei Anregung von beispielsweise 1 kHz, ist: Amplitude (und Phase) des Heizers zeigen bei einem (großen) Gasvolumen (d = 50 µm, b = 20 µm) eine Abhängigkeit vom Gasdruck und ist unempfindlich gegenüber Temperaturänderungen. Die Amplitude des Heizers zeigt bei einem (kleinen) Gasvolumen (d = 5 µm, b = 5 µm) eine Abhängigkeit von der Gastemperatur, aber Unempfindlichkeit gegenüber Druckänderungen. Insofern hat sich auch hier bei dem Beispiel gezeigt, dass eine Kombination von zwei oder mehr Sensorzellen mit unterschiedlichen Dimensionierungen (d, b, L), bevorzugterweise auf einem Chip, vorteilhaft ist, und neben der Bestimmung der volumetrischen Wärmekapazität sowie der Wärmeleitfähigkeit es auch ermöglicht, Wide-Range-Sensoren für unterschiedliche Messgrößen Temperatur und Druck zu schaffen. Insofern wird entsprechend Vergleichsbeispielen ein gasunabhängiger Wide-Range-Drucksensor (einige mbar bis einige bar) ohne mechanische Komponenten (Diaphragm) geschaffen. Selbstverständlich lassen sich mit diesem Vergleichsbeispiel hier auch die Gaseigenschaften (Bestimmung der Wärmeleitfähigkeit und der volumetrischen Wärmekapazität) bestimmen. Das ermöglicht im nächsten Schritt die Bestimmung der sogenannten Temperaturleitfähigkeit bzw. Produkt aus Dichte und Wärmeleitfähigkeit. Anmerkung: Temperaturleitfähigkeit ist definiert als Wärmeleitfähigkeit/(Dichte*spezifische Wärmekapazität), d.h. a=k/(ρ*c). Diese Größen sind vorteilhafterweise zur präzisen On-Chip-Signalkompensation bei Strömungssensoren oder Drucksensoren einsetzbar, wie nachfolgend im Zusammenhang mit Fig. 9 noch erläutert werden wird. Darüber hinaus sei an dieser Stelle noch darauf hingewiesen, dass zur Schaffung von unterschiedlich dimensionierten Sensoren auch die Geometrie des Heizers variiert werden kann. Denkbar wären Mäanderformen, wabenförmige Heizstrukturen, Wärmespiegel, wie sie im Zusammenhang mit Fig. 2 erläutert werden.

Entsprechend Ausführungsbeispielen kann eine Anordnung der Sensorik aus Fig. 3 oder 5 mit mikrotechnischen Fertigungsverfahren prozesskompatibel zu weiteren (thermischen) Sensoren erstellt werden und bietet daher ein hohe Integrationsdichte bei Multiparameter-Anwendungen (z. B. Gaszusammensetzung und Flussrate). Durch das geringe Totvolumen kann die Anordnung außerdem hochdynamisch betrieben werden. Entsprechend Ausführungsbeispielen werden freistehende und von Gas umgebende Heizstrukturen durch Opferschichttechnologie (Oberflächenmikromechanik) oder Bulkmikromechanik (Trockenätzen, ...) realisiert. Die so geschaffenen Heizelemente können periodisch durch Joulesche Erwärmung geheizt werden. Gleichzeitig wird die Temperaturantwort des Heizers überwacht. Diese Anordnung erlaubt es, die Sensorstruktur stark zu miniaturisieren. Die Eigenschaften des Gases beeinflussen die resultierende Temperaturantwort des Heizelements (Amplitude, Phasenverschiebung). Aufgrund des geringen Platzbedarfs lassen sich mehrere dieser Sensoren problemlos mit thermischen Strömungssensoren auf Wafer-Level integrieren. Zusätzlich kommen nur thermische Wandlungsprinzipien zum Einsatz. Diese Kombination macht das System einzigartig.

Entsprechend Vergleichsbeispielen sind die Sensoren ausreichend unempfindlich und können im gleichen Frequenzbereich betrieben werden, z.B. wenn sich durch geometrische Parametervariation (Länge, Breite, Schichtdicke, Form, Höhe der Kavität) und/oder unterschiedlicher Materialeigenschaften die resultierende Grenzfrequenz mindestens um den Faktor 10 unterscheidet.

Beispiele zum Verringern der Grenzfrequenz
- Vergrößerung der Länge des Heizers
- Vergrößerung der Höhe der Kavität.

Entsprechend Ausführungsbeispielen wird eine hohe Empfindlichkeit für Wärmeleitfähigkeit bei Anregungen geringer als Grenzfrequenz erreicht, für hohe Empfindlichkeit gegenüber volumetrischer Wärmekapazität sind Anregungen oberhalb der Grenzfrequenz vorteilhaft.
▪ Anhand der Signalamplitude und/oder dem Phasenversatz können Gaseigenschaften abgeleitet werden
▪ Gemessene Gaseigenschaften (k und cv) werden entweder zur direkten Signalkompensation bei thermischen Strömungssensoren eingesetzt und/oder zur Bestimmung von Gaszusammensetzung und Druck

Durch gezielte Variation der Anregung bzw. Anregungsfrequenz werden entsprechend Ausführungsbeispielen bestimmte Sensorgeometrien selektiv zu Messgrößen und unempfindlich gegenüber bestimmten Quereinflüssen. Das gilt für unterschiedliche Sensorgeometrien, aber auch für gleiche Sensorgeometrien. Folglich schafft ein Ausführungsbeispiel ein Sensorsystem umfassend eine Sensorzelle sowie eine Auswertung, wie anhand von Fig. 5 gezeigt werden wird.

Fig. 5 zeigt eine Sensorzelle 10 mit Heizer 12, Kavität 14 und Substrat 16 in Verbindung mit einer Auswertung 50. Die Auswertung 50 dient zur Ansteuerung der Sensorzelle 10 und ist ausgebildet, um die Sensorzelle 10 mit zumindest zwei unterschiedlichen Anregungsvarianten, wie z. B. mittels zwei unterschiedlichen Anregungsfrequenzen, anzusteuern. Beispielsweise kann der Sensor, dem eine Grenzfrequenz für bestimmte Umgebungsbedingungen innewohnt, mit einer Frequenz signifikant unterhalb der Grenzfrequenz, z. B. bei der Hälfte (1/2) der Grenzfrequenz oder einem Drittel (1/3) oder einem Viertel (1/4) der Grenzfrequenz, angeregt werden (erste Messung) und für eine zweite Messung mit einer Anregungsfrequenz signifikant oberhalb der Grenzfrequenz, z. B. beim Zwei- oder Dreifachen der Grenzfrequenz (bevorzugt 3- bis 20-fache Grenzfrequenz). Das heißt also, dass allgemein sich die erste und zweite Messung, die zu unterschiedlichen Zeitpunkten erfolgen, dahingehend unterscheiden, dass unterschiedliche Anregungsfrequenzen verwendet werden, bevorzugterweise eine Anregungsfrequenz kleiner ½ oder kleiner ¼ der Grenzfrequenz und/oder eine Anregungsfrequenz größer dem Dreifachen der Grenzfrequenz. Somit lässt sich also die Sensorzelle 10 bei unterschiedlichen Betriebspunkten betreiben.

Wie anhand von Fig. 6a und 6b ersichtlich wird, ermöglicht der Betrieb bei unterschiedlichen Betriebspunkten, dass sich unterschiedliche Empfindlichkeiten für die Wärmeleitfähigkeit und die volumetrische Wärmekapazität ausbilden. Infolgedessen ist die Auswertung 50 dazu ausgebildet, die Wärmeleitfähigkeit mittels der ersten Messung (Betriebspunkt kleiner Grenzfrequenz) zu bestimmen und die volumetrische Wärmekapazität mittels der zweiten Messung (Betriebspunkt größer Grenzfrequenz) zu bestimmen. Zwei exemplarisch gewählte Betriebspunkte bzw. Frequenzen für die zwei Messungen sind in den Diagrammen von Fig. 6a und 6b illustriert. Diese Betriebsweise ist deshalb vorteilhaft, da so mit nur einem Sensor die volumetrische Wärmekapazität und die Wärmeleitfähigkeit unabhängig voneinander bestimmt werden kann. Der Vorteil bei der Variante aus Fig. 3 liegt darin, dass die Messung gleichzeitig erfolgt, während hier bei Fig. 5 die Messung seriell, d. h. also zu unterschiedlichen Zeitpunkten, erfolgt.

Entsprechend Ausführungsbeispielen kann ein Sensor ausreichende Unempfindlichkeiten gegenüber Quereinflüssen bzw. der in der aktuellen Messung nicht zu messenden Gaseigenschaft ausbilden , z.B. wenn er in der ersten Messung in einem ersten Frequenzbereich betrieben werden, der etwa um den Faktor 4 geringer ist als die Grenzfrequenz und für eine zweite Messung in einem zweiten Frequenzbereich betrieben werden, der etwa um den Faktor 4 größer ist als die Grenzfrequenz des Sensorsystems.

Entsprechend Ausführungsbeispielen kann die Anregungsfrequenz zur Bestimmung von Wärmeleitfähigkeit unterhalb der Grenzfrequenz liegen, z. B. kleiner ¼ oder kleiner ½. Entsprechend weiteren Ausführungsbeispielen kann die Anregungsfrequenz zur Bestimmung der volumetrischen Wärmekapazität oberhalb der Grenzfrequenz liegen, z. B. etwa Faktor 3 - 20 oberhalb oder allgemein größer Faktor 2 oder 3. In diesen Bereichen sind die Empfindlichkeiten Sₖ und S_{cv} unterschiedlich groß. Entsprechend Ausführungsbeispielen hängt die Grenzfrequenz von den Dimensionen des Sensors bzw. der Auslegung des Sensors ab. Insofern sind die Zusammenhänge zwischen Sensordimensionierung und Wahl des Betriebspunkts unter der Maßgabe anzuwenden, dass der Betriebspunkt entsprechend Ausführungsbeispielen für die erste und zweite Messung unterschiedlich in Abhängigkeit der Grenzfrequenz gewählt werden soll, wie im Vorherigen erläutert wurde.

Entsprechend Ausführungsbeispielen gilt unabhängig von der Dimensionierung der Strukturen, dass eine hohe Empfindlichkeit gegenüber der Wärmeleitfähigkeit bei geringen Frequenzen erreicht wird. Die Frequenz kann entsprechend Ausführungsbeispielen auch f = 0 sein, was dem DC-Betrieb entspricht. Insofern liegt die Anregungsfrequenz in einem Bereich von f ≥ 0, d.h. z.B. nahe Null. Entsprechend Ausführungsbeispielen sind die Anregungsfrequenzen unterschiedlich, d. h. betragsmäßig unterschiedlich. Entsprechend Ausführungsbeispielen kann bei hohen Frequenzen die Struktur unempfindlich gegenüber der Wärmeleitfähigkeit sein (sₖ gegen 0). Entsprechend weiteren Ausführungsbeispielen kann die Empfindlichkeit gegenüber volumetrischer Wärmekapazität ein lokales Maximum aufweisen. $\partial {S}_{cv} / \partial cv = 0$

Aus der Literatur ist bekannt, dass eine gezielte Optimierung (sowohl der Geometrie als auch der Frequenz) auf Basis des Parameter-Modells nicht möglich ist. Daraus leitet sich die Suche eines geeigneten Optimums für den Arbeitspunkt der Sensoranordnung ab.

Bezüglich der Bestimmung der Sensitivitäten für die Wärmeleitfähigkeit und die volumetrische Wärmekapazität sei auf obige Formel, die im Zusammenhang mit Fig. 3 erläutert wurde, hingewiesen.

Entsprechend weiteren Ausführungsbeispielen kann statt einer Variation der Anregungsfrequenzen mit zwei fixen Frequenzen auch der Sensor 10 mit einem variierenden Signal, z. B. einem Dirac-Signal, angeregt werden und dann die Auswertung bei unterschiedlichen Frequenzen erfolgen, bei welchen sich die entsprechenden Sensitivitäten für die Wärmeleitfähigkeit und die volumetrische Wärmekapazität ausbilden. Das Prinzip wurde im Zusammenhang mit Fig. 3 erläutert (siehe Punkt "Zur Auswertung"), ist aber entsprechend Ausführungsbeispielen auf die Ausführung aus Fig. 5 übertragbar. Mögliche Varianten für eine Anregungssignal wurde im Zusammenhang mit Fig. 3 erläutert (siehe Punkt "Zur Anregung"). Bezüglich der Abhängigkeit zu den Sensordimensionen sei auf die Dimensionierungsvarianten aus Fig. 3 verwiesen.

Entsprechend Ausführungsbeispielen kann als Sensor 10 einer der Sensoren aus den Fig. 2a-q verwendet werden.

Anwendungen für das Sensorsystem aus Fig. 5 werden im Zusammenhang mit Fig. 9a bzw. insbesondere im Zusammenhang mit Fig. 9b-d erläutert. Beispielsweise kann das Sensorsystem aus Fig. 5 bzw. das Betriebsverfahren zum Betreiben eines Sensors, wie es im Zusammenhang mit Fig. 5 erläutert ist, für Wide-Range-Sensoren, wie z. B. Wide-Range-Drucksensoren, eingesetzt werden. Ferner ist entsprechend weiteren Ausführungsbeispielen der Einsatz des Sensorsystems aus Fig. 5 bzw. des entsprechenden Betriebsverfahrens für Anwendungen zur Flussmessung (kompensierter Flusssensor) oder für kompensierte Drucksensoren einsetzbar.

Bezüglich der Anregung sei ferner angemerkt, dass beispielsweise ein abwechselndes Springen zwischen zwei Frequenzen zum Erreichen hoher Empfindlichkeit gegenüber Wärmeleitfähigkeit und volumetrische Wärmekapazität (z. B. reines Sinussignal oder Nutzung von Oberwellen) möglich ist. Die mindestens zwei unterschiedlichen Anregungen werden für einen, zwei oder mehreren Sensoren (je nach Aufbau aus Fig. 3 oder Fig. 5) verwendet. Es kommt zu einer modulierten periodischen Anregung mit beiden Auswertefrequenzen für Wärmeleitfähigkeit und volumetrische Wärmekapazität. Mögliche periodische Signalformen neben sinusförmiger Anregung sind Rechtecksignal oder Sägezahnsignal.

Nachfolgend werden Bezug nehmend auf Fig. 2a-o unterschiedliche Sensorzellen erläutert, die bei allen oben genannten Beispielen (Ausführungsbeispielen oder Vergleichsbeispielen) eingesetzt werden können.

Fig. 2a zeigt den bekannten Sensor 10 aus Fig. 1a und 1b mit dem Heizer 12 über der Kavität 14. In Fig. 2b ist ein mäanderförmiger Heizer 12' über der Kavität 14 gezeigt, bei dem Ausführungsbeispiele zeigen, dass durch unterschiedliche Geometrievarianten des Heizers 12' unterschiedliche Dimensionierungen erreicht werden, da der Heizer 12' signifikant länger ist als der Heizer 12. Bei beiden Heizern 12 und 12' handelt es sich um freitragende Strukturen bzw. freitragende Brückenstrukturen, die sich über der Kavität 14 befinden.

Fig. 2c zeigt ebenfalls eine freitragende Brückenstruktur, allerdings mit Zuleitungen, die einen verbreiterten Querschnitt aufweisen. Insofern wird sich ein Temperaturspot des Heizers 12" im Zentrum ausbilden. Ein ähnlicher Temperaturspot bildet sich auch bei dem Heizer 12‴ aus Fig. 2d aus, da hier die Mäanderform insbesondere im Zentrum der Kavität angeordnet ist. Hier ist die Kavität 14' gegenüber der Kavität 14 aus Fig. 2b vergrößert. Alle vorherigen Ausführungsvarianten aus den Fig. 2a, 2b, 2c und 2d hatten gemein, dass die Kavität eine grundsätzlich rechteckige Form hat. Das ist allerdings nicht zwingend notwendig, wie beispielsweise anhand Fig. 2e gezeigt ist.

Fig. 2e zeigt eine Sensorzelle mit einer runden Kavität 14" und einem spiralförmigen Heizer 12ʺʺ. Dieser weist ein flächiges Bild auf.

Wie oben bereits erläutert, kann der Heizer entweder die Form einer freitragenden Brückenstruktur, wie z. B. aus Fig. 2a, 2c oder 2d ersichtlich, aufweisen. Der Heizer besteht beispielsweise aus einem leitfähigen Material, das bei Stromdurchfluss eine entsprechende Joulesche Energie emittiert. Das leitfähige Material, wie z. B. das Metall, formt hierbei die freitragende Struktur. Entsprechend weiteren Ausführungsbeispielen wäre es auch denkbar, dass zusätzliche Stützstrukturen, z. B. von einer Membran oder perforierte Membran, vorgesehen sind. Entsprechend Ausführungsbeispielen kann die freitragende Struktur einseitig oder zweiseitig oder allgemein mehrseitig eingespannt sein. Bei einer einseitig eingespannten Struktur kann man auch von einem Heizsteg sprechen.

Entsprechend Ausführungsbeispielen wäre es auch denkbar, dass der Heizsteg bzw. allgemein die freitragende Struktur perforiert ist, wie in Fig. 2g anhand der Struktur 12ʺʺ gezeigt ist. Eine Vergrößerung der perforierten Struktur 12ʺʺ ist in Fig. 2f illustriert. Hier ist zu erkennen, dass sechseckige Öffnungen vorgesehen sind.

Entsprechend weiteren Ausführungsbeispielen können auch zwei Heizer 12a und 12b über einer Kavität 14 angeordnet sein, wie in Fig. 2h zu erkennen ist. Die Heizer können gleich oder unterschiedlich sein. Die hier dargestellten Heizer 12a und 12b sind parallel zueinander und in einer selben beabstandeten Höhe über der Kavität 14 bzw. der Wärmesenke am Boden der Kavität 14 angeordnet. Entsprechend weiteren Ausführungsbeispielen wäre es auch denkbar, dass die zwei Heizer 12a und 12b sich über der Kavität 14 kreuzen, so dass dann die zwei Heizer 12a und 12b in unterschiedlichen Höhen angeordnet sind.

Entsprechend einer weiteren Variante kann je Heizer 12a und 12b eine eigene Kavität 14a und 14b vorgesehen sein, wie anhand von Fig. 2j illustriert ist.

Bei obigen Ausführungsbeispielen wurde davon ausgegangen, dass die Kavität 14 bzw. 14a bzw. 14b oder um genau zu sein der Boden der Kavität als Wärmesenke dient. Insofern ist der Abstand maßgeblich für das Schwingungsverhalten, so dass über diesen Abstand die einzelnen Sensorzellen unterschiedlich dimensioniert werden können. Entsprechend weiteren Ausführungsbeispielen wäre es auch denkbar, dass eine alternative oder zusätzliche Wärmesenke neben dem Heizer eingebracht wird, was anhand von Fig. 2k gezeigt ist. Neben dem Heizer 12, der über der Kavität 14 angeordnet ist, ist noch eine weitere Wärmesenke, z. B. aus Metall, vorgesehen, die hier mit dem Bezugszeichen 13 versehen ist. Auch können zwei Wärmesenken durch zwei den Heizer 12a umschließende Substrate 16a und 16b gebildet sein. Zwischen den zwei Substraten 16a und 16b wird eine Kavität ausgebildet, in welcher der Heizer 12 positioniert ist. Die Verwendung von einer Vielzahl von Wärmesenken 16a, 16c und 16d ist in Fig. 2m gezeigt. Bei diesem Ausführungsbeispiel ist auf einem Substrat 16 eine Abstandshalterschicht 17 aufgebracht, die eine Aussparung unter einem Heizer 12 aufweist, so dass unter dem Heizer 12 eine Kavität gebildet ist. Seitlich neben dem Heizer 12 ist in derselben Ebene des Heizers 12 jeweils eine Wärmesenke 16c1 und 16c2 vorgesehen.

Fig. 2n zeigt eine weitere Variante. Hier sind mehrere Heizer 12* als parallele, von einem Substrat 16 beabstandete Strukturen/Heizstege auf die Substratoberfläche des Substrats 16 aufgebracht. Der Heizer des Substrats 12* ist als Heizsteg ausgeführt mit einem Fußpunkt, der gegenüber dem Heizsteg signifikant verbreitert ist. Das hat insbesondere fertigungstechnische Hintergründe. Ein weiterer, ähnlich hergestellter und ausgebildeter Heizer ist in Fig. 2o gezeigt. Hier ist der Heizsteg wiederum mit dem Bezugszeichen 12* versehen. Die Varianten aus Fig. 2n und 2o stellen sogenannte Oberflächenmikromechaniken dar.

Zusammenfassend ist also festzustellen, dass unterschiedlichste Ausführungen Verwendung finden können, z. B. Wabenstrukturen, Membran (mit/ohne Löcher), zusätzliche Elemente zur aktiven Wärmeübertragung an das Messgas (z. B. Aluminium), mäanderförmige Anordnungen, etc. Nachfolgend wird auf die mögliche Anordnung eines optionalen Detektors eingegangen.

Fig. 2p zeigt den Heizer 12 zusammen mit dem Detektor 18. Beide sind nebeneinander in der gleichen Ebene, d. h. gleich beabstandet, über der Kavität 14 angeordnet.

Fig. 2q zeigt den Heizer 12, auf welchem mittels einer Isolationsschicht getrennt der Detektor 18 angeordnet ist. Die Isolationsschicht ist mit dem Bezugszeichen 18i versehen. Vorteilhaft ist hier, dass die Anregung und die Detektion nah beieinander erfolgt, wobei trotzdem Anregung und Detektion getrennt sind. Hierfür gibt es z.B. folgende Detektionsvarianten entsprechend Ausführungsbeispielen:
- Widerstands-Detektor wird über Heizer gestapelt und durch Isolationsschicht getrennt
- Widerstands-Detektor wird neben Heizer angeordnet (Parallel, Umrundung des Heizers,...)
- Thermoelemente können analog zu Widerstands-Detektoren verwendet werden.

Alternativ kann der Heizer selber als Detektor, z.B. durch Auswertung eines elektrischen Antwortsignals verwendet werden. D.h., dass entsprechend weiteren Ausführungsbeispielen die Anregung und die Detektion mit dem gleichen Element (Joulesche Erwärmung des Heizers und resistive Auswertung des Temperatursignals) erfolgen kann. Diese Variante ist nicht dargestellt.

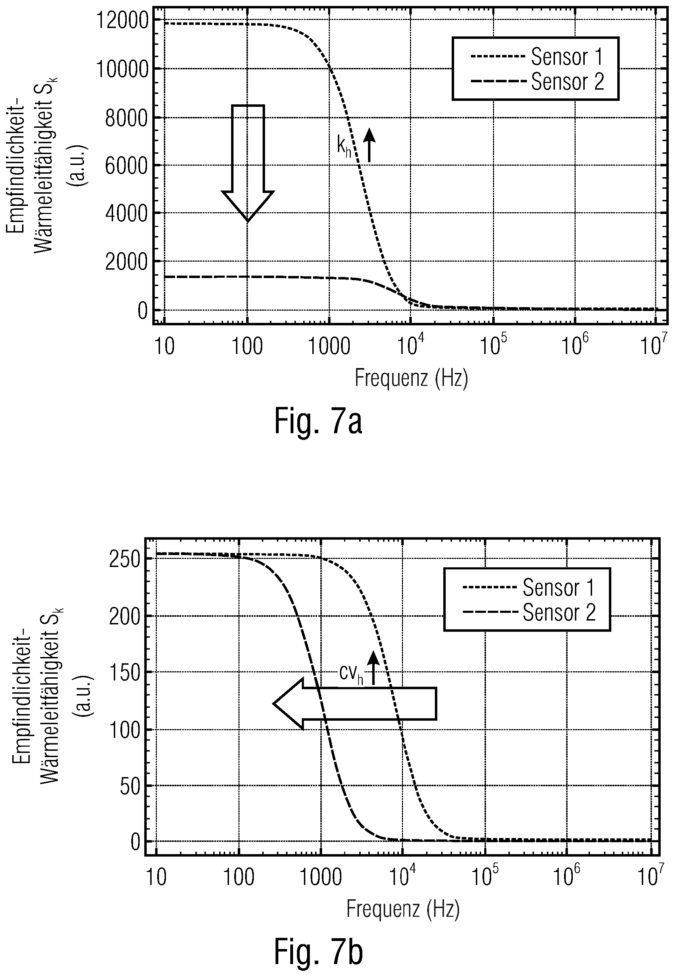

In Fig. 7 werden Möglichkeiten aufgezeigt die frequenzabhängige Empfindlichkeit Sₖ mit Hilfe der Materialeigenschaften der Heizstruktur zu verändern.

Bezug nehmend auf Fig. 7a, die die Empfindlichkeit von kh über die Frequenz und Fig. 7b, die die Empfindlichkeit von cvh über die Frequenz für jeweils zwei unterschiedliche Sensoren zeigt, wird die Auslegung von einem bzw. zwei Sensoren bei einer Implementierung nach Fig. 3 oder bei der Implementierung nach Fig. 5 erläutert.

In Fig. 7 a ist die Auswirkung der Wärmeleitfähigkeit des Sensorsignals auf die frequenzabhängige Empfindlichkeit Sₖ gezeigt. Beide Sensoren sind baugleich, jedoch hat die Heizstruktur von Sensor 1 eine geringere Wärmeleitfähigkeit kₕ als Sensor 2. In Fig. 7 b ist die Auswirkung der Wärmeleitfähigkeit des Sensorsignals auf die frequenzabhängige Empfindlichkeit Sₖ gezeigt. Beide Sensoren sind baugleich, jedoch hat die Heizstruktur von Sensor 1 eine geringere volumetrische Wärmekapazität cvₕ als Sensor 2.

Sensor 1 bzw. Messung M1 soll empfindlich gegenüber k sein, während Sensor 2 bzw. Messung M2 unempfindlich gegenüber k sein soll. Hierfür bieten sich zwei unterschiedliche Lösungen an, nämlich:
a) Lösung bei geringen Frequenzen (f << f_{Grenz} bzw. f → 0)
b) Lösung bei höheren Frequenzen im Bereich der Grenzfrequenz (0,5 * f < f_{Grenz}).

Für a) ergeben sich die drei folgenden Optimierungsmöglichkeiten:
▪ Optimiere Verhältnis der Kavitäten: d₁/d₂ > 20
▪ Optimiere Verhältnis der Wärmeleitfähigkeiten der Heizer: kₕ₁/kₕ₂ < 0,05
▪ Optimiere Verhältnis von Produkt aus Schichtdicke und Breite der Heizer: (b₁*h₁)/(b₂*h₂) < 0,2 (Voraussetzung: gleiche Wärmeübertragungsfläche an das Gas).

Für Lösung b) ergibt sich folgende Optimierungsmöglichkeiten:
Optimiere Verhältnis von Produkt aus volumetrischen Wärmekapazität und Höhe des Heizers: (cvₕ₁*h₁)/(cvₕ₂*h₂) < 0,25

An dieser Stelle sei angemerkt, dass oben genannte Optimierungsmöglichkeiten jeweils als einzelne Ausführungsbeispiele zu verstehen sind, so dass auch weitere Optimierungsvarianten entsprechend weiteren Ausführungsbeispielen denkbar sind.

Entsprechend Ausführungsbeispielen kann eine Geometrieanpassung auf die Gase erfolgen. Je höher k_{gas} desto größer kann die Kavität ausfallen. Eine Verdopplung von k_{gas} führt zur Vervierfachung von d. Je geringer k_{gas} und je höher cv_{gas} ist desto geringer kann die Frequenz gewählt werden.

Ausgehend von Anforderungen, dass mittels Sensor 1 bzw. Messung M1 eine ausreichende Empfindlichkeit gegenüber cv möglich sein soll und dass mittels Sensor 2 bzw. Messung M2 eine ausreichende Unempfindlichkeit gegenüber cv erreicht werden soll, kann folgende Lösung bei höheren Frequenzen im Bereich der Grenzfrequenz gewählt werden.

In der Zusammenschau der beiden Anwendungen unter Berücksichtigung der Lehren, die aus den Fig. 7a und 7b zu ermitteln sind, ist entsprechend Ausführungsbeispielen festzustellen, dass die Wärmeleitfähigkeit bevorzugterweise unterhalb der Grenzfrequenz ausgewertet wird, während die volumetrische Wärmekapazität oberhalb der Grenzfrequenz ausgewertet wird.

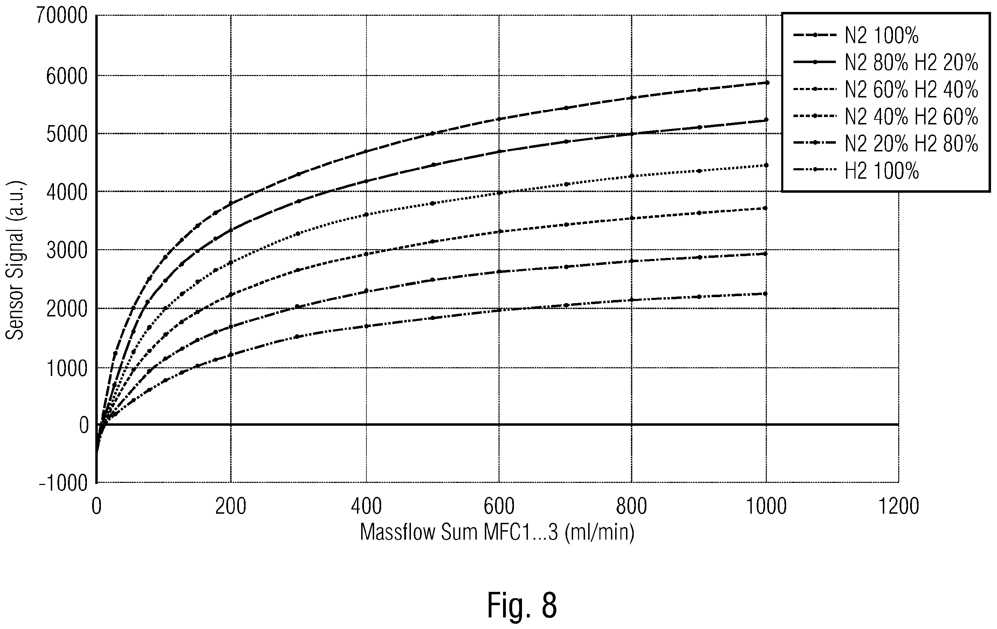

Wie bereits oben erwähnt, hat die Gaszusammensetzung eine Auswirkung auf sämtliche Messungen, und damit auch auf das Ausgangssignal bei thermischen Strömungssensoren, die anhand von Fig. 8 ersichtlich wird.

Fig. 8 zeigt ein Sensorsignal in Abhängigkeit einer gemessenen Flussrate für unterschiedliche Gaszusammensetzungen. Hier kommt es zur Ausbildung einer Kennlinien-Schar. Hierin wird der Bedarf ersichtlich, dass die relevanten Gasparameter in unmittelbarer Nähe zum thermischen Strömungssensor bevorzugterweise ermittelt werden, um die Kennlinie bei Strömungssensoren zu bestimmen. Gaszusammensetzung lässt sich einfach und effektiv über die Varianten aus Fig. 3 bzw. 5 unter Berücksichtigung der obigen Lehre ermitteln.

Diese mikrotechnischen Sensoren aus Fig. 3 und 5 können entsprechend Ausführungsbeispielen entweder zur Signalkompensation bei thermischen Strömungssensoren mit wechselnden Gasmedien und Betriebsparametern (Druck, Temperatur) genutzt werden, bieten aber auch die Möglichkeit als einzelne Sensoren zur Bestimmung der volumetrischen Wärmekapazität, Wärmeleitfähigkeit, Temperatur und Druck eingesetzt zu werden.

Hieraus ergibt sich also die Anwendung eines inline-fähigen Flusssensors mit der Möglichkeit zur Signalkompensation. Fig. 9a zeigt einen Flusssensor 70 mit dem eigentlichen Flusssensor 72 in Kombination mit einer Sensoranordnung 1 umfassend die zwei Sensorchips 10a und 10b. Der Sensor 1 hat mehrere Gasparameter-empfindliche Sensoren und befindet sich in einer Kavität des Chips 72, nämlich in einem strömungsberuhigten Bereich. Auf der Oberfläche des Sensors 70 ist ein thermischer Flusssensor mit gelöcherter Membran zum Gasaustausch vorgesehen.

Die Funktionalität Übertragen auf die Variante des Sensors aus Fig. 5 ist schematisch in Fig. 9b dargestellt. Fig. 9b zeigt die zwei Sensoren 10 und 72. Mittels des Sensors (erste Messung M1) wird k eines bekannten Gasgemisches 3 bestimmt. Ferner wird mit demselben Sensor (in zweite Messung M2) für dasselbe Gasgemisch 3 ρ * c bestimmt. Diese zwei bestimmten Parameter können zur Kompensation einer Auswerteeinrichtung des Flusssensors 72 übergeben werden, der eine Flussrate des Gasgemisches 3 bestimmt. Die Kompensation kann beispielsweise mittels einer Lookup-Table erfolgen, so dass dann eine kompensierte Flussrate bestimmt wird.

Für ein bekanntes Gasgemisch 3 (Wärmeleitfähigkeit und volumetrische Wärmekapazität bei Referenztemperatur und Referenzdruck sind bekannt) kann sich folgendes Verfahren einstellen:
- Auswertung der Ausgangssignale (Amplitude der Temperaturantwort) von Messung M1, die proportional zu der Wärmeleitfähigkeit ist. Die Wärmeleitfähigkeit ist abhängig von der Temperatur und dient zur Bestimmung der mittleren Gastemperatur.
- Erhalten des Ausgangssignals vom Sensor aus Messung M1 wird zur Kompensation der Ausgangssignale von Sensorsignale 10b (Amplitude) genutzt. Ausgangssignal vom Sensor für Messung M2 ist abhängig von der volumetrischen Wärmekapazität. Durch Kompensation kann Dichte bestimmt werden, was zur Bestimmung von Druck dient.

Entsprechend weiteren Ausführungsbeispielen kann basierend auf den Sensorwerten der Messung M1 die Gastemperatur T = f(k_{gas}) bestimmt werden, mittels des Sensorsignals der Messung M2 der Druck p = f(cv_{gas}). Hierbei kann in beiden Varianten eine Lookup-Table verwendet werden. An dieser Stelle sei angemerkt, dass es nicht zwingend erforderlich ist, dass das Gasgemisch bekannt ist (vergleiche Fig. 9c).

Beispielsweise kann unter Verwendung eines weiteren Sensors 75, nämlich eines Temperatursensors, auch basierend auf einem unbekannten Gemisch 3* die Gaszusammensetzung basierend auf dem Sensorsignal der Messung M1 bestimmt werden. Unter Kenntnis dieser Gaszusammensetzung Vol. % = f(k_{gas}) kann unter Verwendung des Sensorsignals der Messung M2 eine korrigierte Flussrate bestimmt werden, wie oben bereits erläutert (unter Verwendung des Flusssensors 72).

Wie anhand von Fig. 9d gezeigt ist, ist der Temperatursensor nicht zwingend notwendig, da auch auf Basis des unbekannten Gasgemisches 3* die Bestimmung der zwei Werte k und ρc über die zwei Messungen ausreicht, um die Flussrate des Flusssensors 72 zu kompensieren. Die Besonderheit hierin liegt, dass Temperatur, Druck und Gaszusammensetzung nicht direkt bestimmt werden, wobei diese aber auch nicht für die absolute Signalkompensation notwendig sind.

Obige Erläuterungen haben gezeigt, dass ein weiteres Vergleichsbeispiel sich auf einen Flusssensor mit einer Sensoranordnung aus Fig. 3 bezieht. Hierbei hat der Sensor 10a beispielsweise eine hohe Empfindlichkeit für die Wärmeleitfähigkeit und der Sensor 10b eine hohe Empfindlichkeit für die volumetrische Wärmekapazität. Entsprechend einem Ausführungsbeispiel kann auch der Sensor 10 mit der Auswertung 50 aus Fig. 5 verwendet werden, da entsprechend im Betrieb dieselben Parameter über zwei Messungen (Messung M1 und Messung M2, z.B. zu zwei verschiedenen / aufeinanderfolgenden Zeitpunkten t1, t2) bestimmbar sind.

Ein weiteres Ausführungsbeispiel bezieht sich auf einen Drucksensor, der einen kompensierten Druck unter Kenntnis der Parameter k und ρ * c bestimmt.

An dieser Stelle sei angemerkt, dass die Wärmleitfähigkeit bevorzugterweise unterhalb der Grenzfrequenz ausgewertet wird, wie anhand von Fig. 7a und 7b zu erkennen ist.

Ein weiteres Ausführungsbeispiel bezieht sich auf ein Verfahren zum Betreiben der Sensoranordnung. Hierbei kann entsprechend Ausführungsbeispielen der Arbeitspunkt bestimmt werden. Ein Verfahren zur Bestimmung der optimalen Sensorkonfiguration kann wie folgt gestaltet sein:
Um den Sensor / die Sensoranordnung beispielsweise für einen universellen Messbereich zu konfigurieren, können entsprechend Ausführungsbeispielen die Arbeitspunkte mit einem selbstjustierenden Verfahren gesucht werden:
▪ Sensorgeometrie: Erstellung und Variation der Strukturen für den Betrieb mit gleicher Frequenz
▪ Sensor-Betrieb: Frequenzscan zur Bestimmung des gasabhängigen AP (auch von Druck/Temperatur abhängig) → Suche des lokalen Maximums für den höchsten Empfindlichkeitsunterschied zwischen Sensorgruppe 1 und 2

Um die optimalen Arbeitspunkte mit der höchsten Empfindlichkeit gegenüber der Messgröße (Wärmeleitfähigkeit oder volumetrische Wärmekapazität) für eine Sensoranordnung innerhalb des mehr-dimensionalen Parameter-Feldes zu bestimmen, müssen Änderungen der Wärmeleitfähigkeit (z. B. Temperaturvariationen, Gaszusammensetzung) und/oder volumetrischen Wärmekapazität (z. B. Druck, Gaszusammensetzung) hervorgerufen werden: Das kann für eine konfigurierte Sensoranordnung auf einem Kalibrier-Messplatz erfolgen. Jedoch auch im nicht-optimalen Arbeitspunkt kann eine Bestimmung der Messgrößen erfolgen, d. h. die Sensoranordnung ist auch im nicht-kalibrierten Zustand verwendbar.

Entsprechend Ausführungsbeispielen weist der Sensorchip, z. B. der Sensorchip aus Fig. 5 oder auch der Sensorchip aus Fig. 3, eine Auswertung auf. Bevorzugterweise werden die ein oder mehreren Sensoren auf einem gemeinsamen Chip hergestellt. Je nach Kompatibilität zu den Fertigungsprozessen mit thermischen Strömungssensoren kann auch der Strömungssensor auf demselben Chip hergestellt werden. Entsprechend einem weiteren Ausführungsbeispiel kann auch der ASIC auf demselben Chip hergestellt werden. Der ASIC oder bzw. allgemein die Auswerteelektronik ist ausgebildet, die Kombination der Signale mehrerer Sensoren zur dynamischen On-Chip-Signalkompensation von thermischen Strömungssensoren leicht zu verarbeiten. Somit wird also ein stark miniaturisierter Sensor mit hoher Dynamik und Potenzial zur monolithischen Integration geschaffen.

Entsprechend Ausführungsbeispielen sind ein oder mehrere Sensoren oder ein und/oder mehrere Sensoren zusammen mit der Auswertung monolithisch integriert. Hierbei kann dann entweder nur der Sensor zur Bestimmung der Gasparameter geschaffen werden oder dieser Sensor auch durch Drucksensoren oder Strömungssensoren erweitert werden.

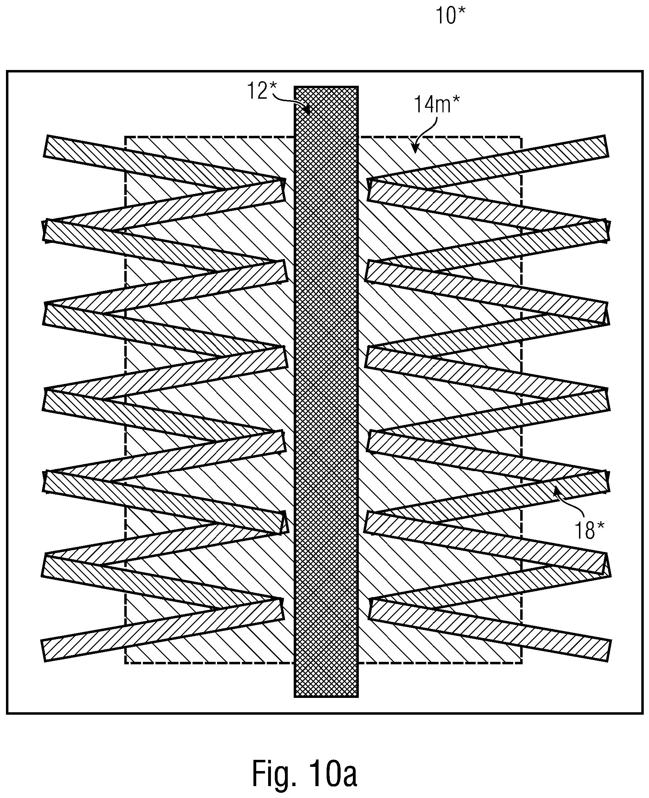

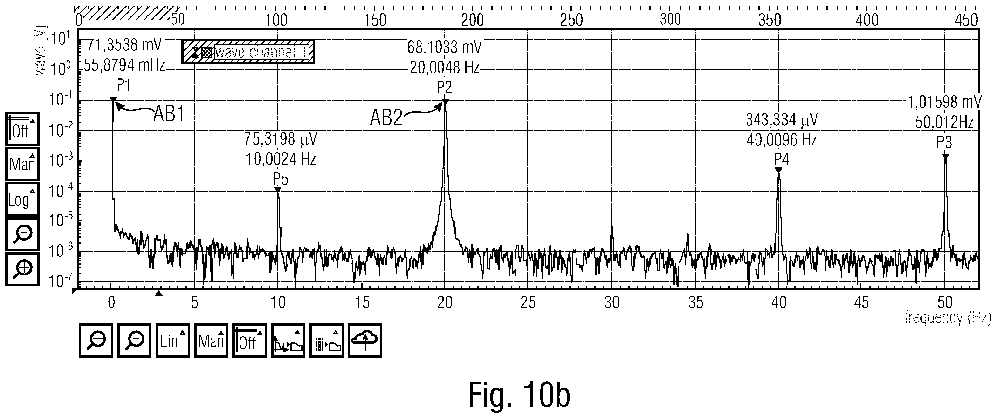

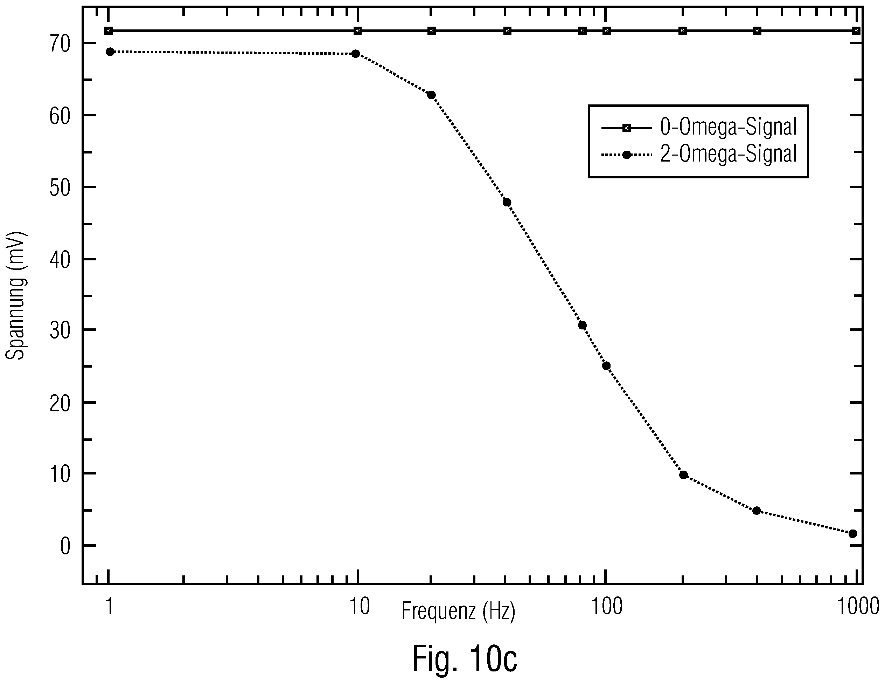

Bezugnehmend auf Fig. 10a, 10b und 10c wird ein erweitertes Ausführungsbeispiel im Zusammenhang mit der Auswertung erläutert. Fig. 10a4zeigt hierbei ein potenziell einzusetzendes Heizelement 10* im Layout. Diesbezüglich sei angemerkt, dass dieses Layout gemäß Ausführungsbeispielen auch für obige Ausführungsbeispiele Anwendung finden kann. Fig. 9b zeigt ein Diagramm, erhalten über eine FFT zur Illustration des Ausführungsbeispiels. Fig. 10c zeigt ein Diagramm (Spannung über Frequenz) zur Illustration von zwei Auswertesignalen bzw. Auswertefrequenzen.

Fig. 10a zeigt ein Layout 10* mit einem oder mehreren Heizern 12* (z. B. Metalldrähte), die auf einer optionalen Membran 14m (die die Kavität bedeckt) angeordnet sein können, und Thermoelemente 18* zur Temperatur-Detektion des Heizelements 12*. In dem Metalldraht wird ein periodischer Strom mit der Kreisfrequenz Ω eingespeist: I(t) = I0 x cos(Ωt). Die in den Heizdraht eingespeiste thermische Leistung ist dann: P(t) I² x R = I(t)² x R = (I0² x R/2 )x (1+cos(2Ωt)), wobei R der elektrische Widerstand des Heizers 12* ist. Durch die eingespeiste Leistung ändert sich die Temperatur des Heizers mit der gleichen Frequenz wie das Leistungssignal: T(t) = T0(t) + ΔT x cos (2Ωt+Φ). Die Amplitude ΔT und die Phasenverschiebung Φ gegenüber der Einspeiseleistung ist abhängig von der Wärmeleitfähigkeit des Materials und der Frequenz Ω. T0 ist die Nulllage der Temperaturschwingung und abhängig von Leistung und Ankopplung der Probe an die Umgebung. Die Temperaturoszillation des Heizdrahts führt zu einer Widerstandsoszillation desselben. Diese Erläuterung der Einkopplung bzw. Verursachung der thermischen Schwingung ist auf obige Ausführungsbeispiele anzuwenden.

Ausgehend von einer so thermisch angeregten Schwingung kann das Heizelement, hier das Heizelement 12* mit nur einer Frequenz (z. B. 1 kHz) angeregt werden. Bei diesem Ausführungsbeispiel wird dann die Auswertefrequenz an zwei Punkten, z. B. bei der 0. und der 2. harmonischen ausgewertet. Die 0. harmonische Schwingung ist auch als DC-Signal bezeichnet. Die 2. als 2-Omega-Signal. Diese Auswertung mittels einer FFT-Analyse ist anhand von Fig. 10b illustriert. Bei der FFT-Analyse bilden sich verschiedene harmonische Schwingungen aus. Interessant sind vor allem das 0-Omega-Signal und das 2-Omega-Signal, die hier mit AB1 und AB2 markiert sind. AB1 stellt das 0-Omega-Signal bzw. DC-Signal dar und gilt als Maß für T0. AB2 stellt das 2-Omega-Signal dar und gilt als Maß für ΔT.

Hieraus ergibt sich dann das Temperatursignal T(t) = T0 (t) + ΔT x cos(2Ωt + Φ). Entsprechend weiteren Ausführungsbeispielen kann auch ein anderes harmonisches Signal oder ein anderes Frequenzsignal verwendet werden, wie z. B. das 1-Omega-Signal.. An dieser Stelle sei angemerkt, dass bei den hier in Fig. 10b dargestellten Ausführungsbeispielen eine Anregung von 10 Hz verwendet wurde. Die Auswertefrequenzen sind 0 Hz und 20 Hz in diesem Ausführungsbeispiel.

In Fig. 10c ist dann das 0-Omega-Signal sowie das 2-Omega-Signal über den Frequenzbereich dargestellt. Ausgehend von diesem Diagramm kann das 0-Omega-Signal (DC-Signal) als Maß für die Wärmeleitfähigkeit verwendet werden, während das 2-Omega-Signal bei höheren Frequenzen ein Maß für Temperaturleitfähigkeit und volumetrische Wärmekapazität darstellt.

Obiges Ausführungsbeispiel hat also gezeigt, dass auch als Auswertefrequenz eine 0 Hz-Frequenz verwendet werden kann, wenn zwei Auswertefrequenzen Verwendung finden sollen. Dieses Vorgehen hat sich bei Erstmessungen als nützlich herausgestellt, um bei nur einer Anregungsfrequenz eine hohe Empfindlichkeit gegenüber der Wärmeleitfähigkeit (DC-Komponente) als auch eine hohe Empfindlichkeit gegenüber der volumetrischen Wärmekapazität zu erreichen.

Entsprechend einem Ausführungsbeispiel kann die Auswertung bei den zwei Auswertefrequenzen mittels einer FFT-Analyse erfolgen. Entsprechend Ausführungsbeispielen kann die FFT-Analyse der harmonischen Temperatursignale (z. B. 2x Anregungsfrequenz und 0x Anregungsfrequenz (DC-Signal) durchgeführt werden). Entsprechend einem weiteren Ausführungsbeispiel misst der Detektor das DC-Signal, während ein weiterer Detektor die harmonischen Signale misst. Eine Mehrzahl an Detektoren in Verbindung mit einem Heizelement ist in Fig. 10a dargestellt, wobei das nur exemplarisch ist.

Ein Vergleichsbeispiel schafft eine Sensoranordnung mit mindestens zwei stark miniaturisierten Sensoren mit thermischen Wirkprinzipien zur Bestimmung einer einzelnen Gaseigenschaft (volumetrische Wärmekapazität cv (Produkt aus Dichte und spezifische Wärmekapazität) oder Wärmeleitfähigkeit k). Diese thermischen Sensoren werden so ausgelegt, dass mindestens ein Bauteil eine hohe Empfindlichkeit gegenüber einer Gaseigenschaft aufweist, während mindestens ein anderes Bauteil eine hohe Empfindlichkeit gegenüber einer anderen Gaseigenschaft besitzt. Die Herausforderung besteht bei der Erzeugung einer gaseigenschaftssensitiven Struktur bei gleichzeitiger Minimierung der Querempfindlichkeiten. Wie bereits erwähnt, sind die Gaseigenschaften nicht nur abhängig von der Zusammensetzung, sondern auch von der Temperatur und dem Druck. Dieser Einfluss ist jedoch unterschiedlich stark ausgeprägt und kann dazu genutzt werden, mehrere kombinierte Gaseigenschaftssensoren indirekt zur Ermittlung von Druck und Temperatur einzusetzen. Geringe Druckänderungen (Δp < 10 bar) führen in erster Näherung nur zu Änderung bei der Gasdichte. Variationen bei der Temperatur (ΔT < 50 K) wirken sich hingegen in erster Näherung auf die Dichte und Wärmeleitfähigkeit aus. Die spezifische Wärmekapazität bleibt hingegen nahezu unbeeinflusst von Druck- und Temperaturänderungen.

Beschreibung von oben Benutzten Variablen:
- Dimensionen des Heizers: L, b, h
- Höhe der Kavität: d
- Materialeigenschaften des Heizers: cvₕ, kₕ
- Gaseigenschaften k, cv bzw. für bessere Eindeutigkeit k_{gas}, cv_{gas}
- Empfindlichkeiten gegenüber Gaseigenschaften Sₖ, S_{cv}
- Breite der effektiven Wärmeübertragungsfläche an das Gas b_{gas}
- Grenzfrequenzen f_{Grenz} bzw. f_{Grenz,S1}

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar. Einige oder alle der Verfahrensschritte können durch einen Hardware-Apparat (oder unter Verwendung eine Hardware-Apparats), wie zum Beispiel einen Mikroprozessor, einen programmierbaren Computer oder eine elektronische Schaltung. Bei einigen Ausführungsbeispielen können einige oder mehrere der wichtigsten Verfahrensschritte durch einen solchen Apparat ausgeführt werden.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einem programmierbaren Computersystem derart zusammenwirken können oder zusammenwirken, dass das jeweilige Verfahren durchgeführt wird. Deshalb kann das digitale Speichermedium computerlesbar sein.

Manche Ausführungsbeispiele gemäß der Erfindung umfassen also einen Datenträger, der elektronisch lesbare Steuersignale aufweist, die in der Lage sind, mit einem programmierbaren Computersystem derart zusammenzuwirken, dass eines der hierin beschriebenen Verfahren durchgeführt wird.

Allgemein können Ausführungsbeispiele der vorliegenden Erfindung als Computerprogrammprodukt mit einem Programmcode implementiert sein, wobei der Programmcode dahin gehend wirksam ist, eines der Verfahren durchzuführen, wenn das Computerprogrammprodukt auf einem Computer abläuft.

Der Programmcode kann beispielsweise auch auf einem maschinenlesbaren Träger gespeichert sein.

Andere Ausführungsbeispiele umfassen das Computerprogramm zum Durchführen eines der hierin beschriebenen Verfahren, wobei das Computerprogramm auf einem maschinenlesbaren Träger gespeichert ist. Mit anderen Worten ist ein Ausführungsbeispiel des erfindungsgemäßen Verfahrens somit ein Computerprogramm, das einen Programmcode zum Durchführen eines der hierin beschriebenen Verfahren aufweist, wenn das Computerprogramm auf einem Computer abläuft.

Ein weiteres Ausführungsbeispiel der erfindungsgemäßen Verfahren ist somit ein Datenträger (oder ein digitales Speichermedium oder ein computerlesbares Medium), auf dem das Computerprogramm zum Durchführen eines der hierin beschriebenen Verfahren aufgezeichnet ist.

Ein weiteres Ausführungsbeispiel des erfindungsgemäßen Verfahrens ist somit ein Datenstrom oder eine Sequenz von Signalen, der bzw. die das Computerprogramm zum Durchführen eines der hierin beschriebenen Verfahren darstellt bzw. darstellen. Der Datenstrom oder die Sequenz von Signalen kann bzw. können beispielsweise dahin gehend konfiguriert sein, über eine Datenkommunikationsverbindung, beispielsweise über das Internet, transferiert zu werden.

Ein weiteres Ausführungsbeispiel umfasst eine Verarbeitungseinrichtung, beispielsweise einen Computer oder ein programmierbares Logikbauelement, die dahin gehend konfiguriert oder angepasst ist, eines der hierin beschriebenen Verfahren durchzuführen.

Ein weiteres Ausführungsbeispiel umfasst einen Computer, auf dem das Computerprogramm zum Durchführen eines der hierin beschriebenen Verfahren installiert ist.

Ein weiteres Ausführungsbeispiel gemäß der Erfindung umfasst eine Vorrichtung oder ein System, die bzw. das ausgelegt ist, um ein Computerprogramm zur Durchführung zumindest eines der hierin beschriebenen Verfahren zu einem Empfänger zu übertragen. Die Übertragung kann beispielsweise elektronisch oder optisch erfolgen. Der Empfänger kann beispielsweise ein Computer, ein Mobilgerät, ein Speichergerät oder eine ähnliche Vorrichtung sein. Die Vorrichtung oder das System kann beispielsweise einen Datei-Server zur Übertragung des Computerprogramms zu dem Empfänger umfassen.

Bei manchen Ausführungsbeispielen kann ein programmierbares Logikbauelement (beispielsweise ein feldprogrammierbares Gatterarray, ein FPGA) dazu verwendet werden, manche oder alle Funktionalitäten der hierin beschriebenen Verfahren durchzuführen. Bei manchen Ausführungsbeispielen kann ein feldprogrammierbares Gatterarray mit einem Mikroprozessor zusammenwirken, um eines der hierin beschriebenen Verfahren durchzuführen. Alternativ kann ein Mikrocontroller (z.B. PSoC: Programmable System on Chip) und/oder Lock-in-Technik eingesetzt werden. Allgemein werden die Verfahren bei einigen Ausführungsbeispielen seitens einer beliebigen Hardwarevorrichtung durchgeführt. Diese kann eine universell einsetzbare Hardware wie ein Computerprozessor (CPU) sein oder für das Verfahren spezifische Hardware, wie beispielsweise ein ASIC.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Sensoranordnung mit mindestens einer Sensorzelle und einer Auswertung (50),
wobei die mindestens eine Sensorzelle thermisch mittels einer Heizers (12) anregbar ist;
wobei die Sensorzelle ausgebildet sind, um in Abhängigkeit von einer Gaseigenschaft eines die Sensorzelle umgebene Gases, insbesondere einer Wärmeleitfähigkeit (k) und/oder volumetrischen Wärmekapazität (cv) und/oder einer Temperatur und/oder einem Druck, ein Schwingungsverhalten auszubilden,
wobei die Sensorzelle mittels mindestens einer Anregungsfrequenz angeregt wird und wobei eine erste Anregungsfrequenz oder eine erste Auswertungsfrequenz für eine erste Messung verwendet wird, und wobei eine zweite Anregungsfrequenz oder Auswertefrequenz für eine zweite Messung (M2) verwendet wird,
wobei sich die erste Anregungsfrequenz von der zweiten Anregungsfrequenz unterscheidet oder wobei sich die erste Auswertefrequenz von der zweiten Auswertefrequenz unterscheidet; und
wobei die Auswertung (50) ausgebildet ist, auf Basis der ersten Messung (M1) eine Wärmeleitfähigkeit (k) des umgebenden Gases und auf Basis der zweiten Messung (M2) eine volumetrische Wärmekapazität (cv) des umgebenden Gases zu bestimmen,
**dadurch gekennzeichnet, dass**
die erste Anregungsfrequenz um mindestens Faktor 2 oder mindestens Faktor 4 kleiner ist als die Grenzfrequenz des Sensors (10) und wobei die zweite Anregungsfrequenz um mindestens Faktor 2 oder mindestens Faktor 4 größer ist als die Grenzfrequenz des Sensors (10); und/oder wobei die erste Auswertefrequenz um mindestens Faktor 2 oder mindestens Faktor 4 kleiner ist als die Grenzfrequenz des Sensors (10) und wobei die zweite Auswertefrequenz um mindestens Faktor 2 oder mindestens Faktor 4 größer ist als die Grenzfrequenz des Sensors (10).

2. Sensoranordnung gemäß Anspruch 1, wobei die erste Anregungsfrequenz und/oder die zweite Anregungsfrequenz größer oder gleich 0Hz sind;
wobei die erste Anregungsfrequenz gleich 0Hz ist und wobei die Anregungsenergie für die erste Messung (M1) größer 0 ist oder wobei die zweite Anregungsfrequenz gleich 0Hz ist und wobei die Anregungsenergie für die zweite Messung (M2) größer 0 ist.

3. Sensoranordnung gemäß Anspruch 1 oder 2, wobei sich die erste Anregungsfrequenz von der zweiten Anregungsfrequenz um mindestens Faktor 2, mindestens Faktor 4 oder mindestens Faktor 8 unterscheidet; und/oder
wobei sich die erste und die zweite Auswertefrequenz um mindestens Faktor 2, mindestens Faktor 4 oder mindestens Faktor 8 unterscheidet.

4. Sensoranordnung gemäß einem der vorherigen Ansprüche, wobei die erste Anregungsfrequenz oder die erste Auswertefrequenz sowie die zweite Anregungsfrequenz oder zweite Auswertefrequenz jeweils durch eine fixe Frequenz definiert ist.

5. Sensoranordnung gemäß einem der vorherigen Ansprüche, wobei die Sensorzelle eine Kavität (14) mit einem Heizer (12), oder eine Wärmesenke mit einem beabstandeten Heizer (12), oder einen von einer Wärmesenke beabstandeten Heizsteg aufweist; und/oder
wobei der Heizer (12) oder der Heizsteg ausgebildet ist, um thermisch zu schwingen und so das Schwingungsverhalten auszubilden; und/oder
wobei der Heizer (12) durch einen Heizsteg oder eine freitragende Struktur oder freitragende Brückenstruktur gebildet ist.

6. Sensoranordnung gemäß einem der vorherigen Ansprüche, wobei die Sensorzelle einen Detektor aufweist, der ausgebildet ist, um das Schwingungsverhalten zu detektieren.

7. Sensoranordnung gemäß einem der vorherigen Ansprüche, wobei die Empfindlichkeit bei der zweiten Messung (M2) für volumetrische Wärmekapazität (cᵥ) um mindestens Faktor 3, mindestens Faktor 4 oder mindestens Faktor 5 höher ist als die Empfindlichkeit bei der ersten Messung (M1) für volumetrische Wärmekapazität (cᵥ); und/oder
wobei die Empfindlichkeit bei der ersten Messung (M1) für Wärmeleitfähigkeit (k) um mindestens Faktor 1,1 oder mindestens Faktor 1,2 höher ist als die Empfindlichkeit bei der zweiten Messung (M2) für Wärmeleitfähigkeit (k).

8. Sensoranordnung gemäß einem der vorherigen Ansprüche, wobei die Auswertung (50) ausgebildet ist, die Sensorzelle periodisch anzuregen.

9. Sensoranordnung gemäß einem der vorherigen Ansprüche, wobei die Sensorzelle mit einer variierenden Anregungsfrequenz, insbesondere einem CHIRP-Signal oder einem DIRAC-Signal angeregt wird.

10. Sensoranordnung gemäß einem der vorherigen Ansprüche, wobei die Auswertung (50) das Schwingungsverhalten des Sensors (10) anhand der dynamischen Temperaturantwort und/oder anhand der Amplitude und/oder anhand der Frequenz und/oder anhand der Phase bestimmt; und/oder
wobei die Auswertung (50) als ASIC implementiert ist, wobei der ASIC in einen Chip oder monolithischen Chip integriert ist, der die Sensorzelle beherbergt.

11. Sensoranordnung gemäß einem der vorherigen Ansprüche, wobei die erste Auswertefrequenz und/oder die zweite Auswertefrequenz größer oder gleich 0Hz ist; und/oder
wobei die Auswertung eine FFT umfasst; oder
wobei die Auswertung eine FFT umfasst und die erste Messung bei einer ersten Auswertefrequenz gleich 0 Hz und/oder die zweite Auswertefrequenz größer 0Hz gleich 0Hz oder bei 1-OMEGA, 2-OMEGA oder 3-OMEGA erfolgt.

12. Flusssensor mit einer Sensoranordnung gemäß einem der vorherigen Ansprüche, wobei der Flusssensor ausgebildet ist, einen Fluss unter Berücksichtigung der bestimmten Wärmeleitfähigkeit (k) und volumetrischen Wärmekapazität (cv) zu bestimmen.

13. Drucksensor umfassend eine Sensoranordnung gemäß einem der Ansprüche 1 bis 11, wobei der Drucksensor ausgebildet ist, den Druck unter Berücksichtigung der volumetrischen Wärmekapazität (cv) und der Wärmeleitfähigkeit (k) zu bestimmen.

14. Verfahren zur Auswertung (50) einer Sensoranordnung gemäß einem der Ansprüche 1 bis 11 mit den folgenden Schritten:
Anregen des Sensors (10) mittels einer Anregungsfrequenz, wobei eine erste Anregungsfrequenz oder eine erste Auswertungsfrequenz für eine erste Messung (M1) verwendet wird, und wobei eine zweite Anregungsfrequenz oder Auswertefrequenz für eine zweite Messung (M2) verwendet wird, wobei sich die erste Anregungsfrequenz von der zweiten Anregungsfrequenz unterscheidet oder wobei sich die erste Auswertefrequenz von der zweiten Auswertefrequenz unterscheidet; und
Bestimmen auf Basis der ersten Messung (M1) eine Wärmeleitfähigkeit (k) des umgebenden Gases und auf Basis der zweiten Messung (M2) eine volumetrische Wärmekapazität (cv) des umgebenden Gases;
**dadurch gekennzeichnet, dass**
die erste Anregungsfrequenz um mindestens Faktor 2 oder mindestens Faktor 4 kleiner ist als die Grenzfrequenz des Sensors (10) und wobei die zweite Anregungsfrequenz um mindestens Faktor 2 oder mindestens Faktor 4 größer ist als die Grenzfrequenz des Sensors (10); und/oder wobei die erste Auswertefrequenz um mindestens Faktor 2 oder mindestens Faktor 4 kleiner ist als die Grenzfrequenz des Sensors (10) und wobei die zweite Auswertefrequenz um mindestens Faktor 2 oder mindestens Faktor 4 größer ist als die Grenzfrequenz des Sensors (10).

15. Computerprogramm zur Durchführung des Verfahrens nach Anspruch 14, wenn das Verfahren auf der Auswertung (50) läuft.

## Claims

1. A sensor arrangement comprising at least one sensor cell and an evaluation (50),
wherein the at least one sensor cell can be excited thermally by means of a heater (12);
wherein the sensor cell is configured to form an oscillation behavior in dependence on a gas property of a gas surrounding the sensor cell, in particular a heat conductivity (k) and/or volume heat capacity (cv) and/or temperature and/or pressure,
wherein the sensor cell is excited by means of at least one excitation frequency, and wherein a first excitation frequency or a first evaluation frequency is used for a first measurement, and wherein a second excitation frequency or evaluation frequency is used for a second measurement (M2),
wherein the first excitation frequency differs from the second excitation frequency, or wherein the first evaluation frequency differs from the second evaluation frequency; and
wherein the evaluation (50) is configured to determine heat conductivity (k) of the surrounding gas based on the first measurement (M1) and volume heat capacity (cv) of the surrounding gas based on the second measurement (M2).
**characterized in that** the first excitation frequency is smaller by at least a factor of 2 or at least a factor of 4 than the cutoff frequency of the sensor (10), and wherein the second excitation frequency is greater by at least a factor of 2 or at least a factor of 4 than the cutoff frequency of the sensor (10); and/or wherein the first evaluation frequency is smaller by at least a factor of 2 or at least a factor of 4 than the cutoff frequency of the sensor (10), and wherein the second evaluation frequency is greater by at least a factor of 2 or at least a factor of 4 than the cutoff frequency of the sensor (10).

2. The sensor arrangement in accordance with claim 1, wherein the first excitation frequency and/or the second excitation frequency are greater than or equaling 0Hz;
wherein the first excitation frequency equals 0Hz and wherein the excitation energy for the first measurement (M1) is greater than 0, or wherein the second excitation frequency equals 0Hz and wherein the excitation energy for the second measurement (M2) is greater than 0.

3. The sensor arrangement in accordance with claim 1 or 2, wherein the first excitation frequency differs from the second excitation frequency by at least a factor of 2, at least a factor of 4 or at least a factor of 8; and/or
wherein the first and the second evaluation frequency differ by at least a factor of 2, at least a factor of 4 or at least a factor of 8.

4. The sensor arrangement in accordance with any of the preceding claims, wherein the first excitation frequency or the first evaluation frequency and the second excitation frequency or second evaluation frequency are each defined by a fixed frequency.

5. The sensor arrangement in accordance with any of the preceding claims, wherein the sensor cell comprises a cavity (14) having a heater (12), or a heat sink having a spaced-apart heater (12), or a heating rib spaced apart from a heat sink; and/or
wherein the heater (12) or heating rib is configured to oscillate thermally and thus form the oscillation behavior; and/or
wherein the heater (12) is formed by a heating rib or a self-supporting structure or self-supporting bridge structure.

6. The sensor arrangement in accordance with any of the preceding claims, wherein the sensor cell comprises a detector configured to detect the oscillation behavior.

7. The sensor arrangement in accordance with any of the preceding claims, wherein the sensitivity to volume heat capacity (cᵥ) in the second measurement (M2) is higher by at least a factor of 3, at least a factor of 4 or at least a factor of 5 than the sensitivity to volume heat capacity (cᵥ) in the first measurement (M1); and/or
wherein the sensitivity to heat conductivity (k) in the first measurement (M1) is higher by at least a factor of 1.1 or at least a factor of 1.2 than the sensitivity to heat conductivity (k) in the second measurement (M2).

8. The sensor arrangement in accordance with any of the preceding claims, wherein the evaluation (50) is configured to periodically excite the sensor cell.

9. The sensor arrangement in accordance with any of the preceding claims, wherein the sensor cell is excited by a varying excitation frequency, in particular a CHIRP signal or DIRAC signal.

10. The sensor arrangement in accordance with any of the preceding claims, wherein the evaluation (50) determines the oscillation behavior of the sensor (10) using the dynamic temperature response and/or using the amplitude and/or using the frequency and/or using the phase; and/or
wherein the evaluation (50) is implemented as an ASIC, wherein the ASIC is integrated in a chip or monolithic chip which accommodates the sensor cell.

11. The sensor arrangement in accordance with any of the preceding claims, wherein the first evaluation frequency and/or the second evaluation frequency are greater than or equaling 0Hz; and/or
wherein the evaluation comprises an FFT; or
wherein the evaluation comprises an FFT and the first measurement takes place at a first evaluation frequency equaling 0Hz and/or the second evaluation frequency is greater than or equaling 0Hz or at 1-OMEGA, 2-OMEGA or 3-OMEGA.

12. A flow sensor comprising a sensor arrangement in accordance with any of the preceding claims, wherein the flow sensor is configured to determine a flow while considering the heat conductivity (k) and volume heat capacity (cv) determined.

13. A pressure sensor comprising a sensor arrangement in accordance with any of claims 1 to 11, wherein the pressure sensor is configured to determine the pressure while considering the volume heat capacity (cv) and heat conductivity (k).

14. A method for evaluating (50) a sensor arrangement in accordance with any of claims 1 to 11, comprising the steps of:
exciting the sensor (10) by means of an excitation frequency, wherein a first excitation frequency or a first evaluation frequency is used for a first measurement (M1), and wherein a second excitation frequency or evaluation frequency is used for a second measurement (M2), wherein the first excitation frequency differs from the second excitation frequency, or wherein the first evaluation frequency differs from the second evaluation frequency; and
determining heat conductivity (k) of the surrounding gas based on the first measurement (M1) and volume heat capacity (cv) of the surrounding gas based on the second measurement (M2);
**characterized in that** the first excitation frequency is smaller by at least a factor of 2 or at least a factor of 4 than the cutoff frequency of the sensor (10), and wherein the second excitation frequency is greater by at least a factor of 2 or at least a factor of 4 than the cutoff frequency of the sensor (10); and/or wherein the first evaluation frequency is smaller by at least a factor of 2 or at least a factor of 4 than the cutoff frequency of the sensor (10), and wherein the second evaluation frequency is greater by at least a factor of 2 or at least a factor of 4 than the cutoff frequency of the sensor (10).

15. A computer program for performing the method in accordance with claim 14 when the method runs on the evaluation (50).

## Revendications

1. Dispositif de capteurs comprenant au moins une cellule de capteur et une unité d'évaluation (50),
dans lequel ladite au moins une cellule de capteur peut être excitée thermiquement au moyen d'un élément chauffant (12) ;
dans lequel ladite au moins une cellule de capteur est conçue pour présenter un comportement oscillatoire en fonction d'une propriété d'un gaz entourant la cellule de capteur, notamment d'une conductivité thermique (k) et/ou d'une capacité thermique volumique (cv) et/ou d'une température et/ou d'une pression,
dans lequel ladite au moins une cellule de capteur est excitée au moyen d'au moins une fréquence d'excitation, et dans lequel une première fréquence d'excitation ou une première fréquence d'évaluation est utilisée pour une première mesure, et dans lequel une deuxième fréquence d'excitation ou une deuxième fréquence d'évaluation est utilisée pour une deuxième mesure (M2),
dans lequel la première fréquence d'excitation diffère de la deuxième fréquence d'excitation ou dans lequel la première fréquence d'évaluation diffère de la deuxième fréquence d'évaluation ; et
dans lequel l'unité d'évaluation (50) est conçue pour déterminer, sur la base de la première mesure (M1), une conductivité thermique (k) du gaz environnant et, sur la base de la deuxième mesure (M2), une capacité thermique volumique (cv) du gaz environnant,
**caractérisé en ce que** la première fréquence d'excitation est inférieure d'au moins un facteur 2 ou d'au moins un facteur 4 à la fréquence limite du capteur (10) et la deuxième fréquence d'excitation est supérieure d'au moins un facteur 2 ou d'au moins un facteur 4 à la fréquence limite du capteur (10) ; et/ou la première fréquence d'évaluation est inférieure d'au moins un facteur 2 ou d'au moins un facteur 4 à la fréquence limite du capteur (10) et la deuxième fréquence d'évaluation est supérieure d'au moins un facteur 2 ou d'au moins un facteur 4 à la fréquence limite du capteur (10).

2. Dispositif de capteurs selon la revendication 1, dans lequel la première fréquence d'excitation et/ou la deuxième fréquence d'excitation sont supérieures ou égales à 0 Hz ;
dans lequel la première fréquence d'excitation est égale à 0 Hz et dans lequel l'énergie d'excitation pour la première mesure (M1) est supérieure à 0, ou dans lequel la deuxième fréquence d'excitation est égale à 0 Hz et dans lequel l'énergie d'excitation pour la deuxième mesure (M2) est supérieure à 0.

3. Dispositif de capteurs selon la revendication 1 ou 2, dans lequel la première fréquence d'excitation diffère de la deuxième fréquence d'excitation d'au moins un facteur 2, d'au moins un facteur 4 ou d'au moins un facteur 8 ; et/ou
dans lequel la première fréquence d'évaluation et la deuxième fréquence d'évaluation diffèrent d'au moins un facteur 2, d'au moins un facteur 4 ou d'au moins un facteur 8.

4. Dispositif de capteurs selon l'une des revendications précédentes, dans lequel la première fréquence d'excitation ou la première fréquence d'évaluation, ainsi que la deuxième fréquence d'excitation ou la deuxième fréquence d'évaluation, sont chacune définies par une fréquence fixe.

5. Dispositif de capteurs selon l'une des revendications précédentes, dans lequel la cellule de capteur comprend une cavité (14) avec un élément chauffant (12), ou un dissipateur thermique avec un élément chauffant (12) espacé, ou une barrette chauffante espacée d'un dissipateur thermique ; et/ou
dans lequel l'élément chauffant (12) ou la barrette chauffante est conçu(e) pour osciller thermiquement et former ainsi le comportement oscillatoire ; et/ou
dans lequel l'élément chauffant (12) est formé par une barrette chauffante, une structure en porte-à-faux ou une structure en pont en porte-à-faux.

6. Dispositif de capteurs selon l'une des revendications précédentes, dans lequel la cellule de capteur comprend un détecteur conçu pour détecter le comportement oscillatoire.

7. Dispositif de capteurs selon l'une des revendications précédentes, dans lequel la sensibilité lors de la deuxième mesure (M2) pour la capacité thermique volumique (cᵥ) est supérieure d'au moins un facteur 3, d'au moins un facteur 4 ou d'au moins un facteur 5 à la sensibilité lors de la première mesure (M1) pour la capacité thermique volumique (cᵥ) ; et/ou
dans lequel la sensibilité lors de la première mesure (M1) pour la conductivité thermique (k) est supérieure d'au moins un facteur 1,1 ou d'au moins un facteur 1,2 à la sensibilité lors de la deuxième mesure (M2) pour la conductivité thermique (k).

8. Dispositif de capteurs selon l'une des revendications précédentes, dans lequel l'unité d'évaluation (50) est conçue pour exciter périodiquement la cellule de capteur.

9. Dispositif de capteurs selon l'une des revendications précédentes, dans lequel la cellule de capteur est excitée avec une fréquence d'excitation variable, en particulier un signal CHIRP ou un signal DIRAC.

10. Dispositif de capteurs selon l'une des revendications précédentes, dans lequel l'unité d'évaluation (50) détermine le comportement oscillatoire du capteur (10) à partir de la réponse thermique dynamique et/ou de l'amplitude et/ou de la fréquence et/ou de la phase ; et/ou
dans lequel l'unité d'évaluation (50) est mise en œuvre sous la forme d'un ASIC, l'ASIC étant intégré dans une puce ou une puce monolithique qui héberge la cellule de capteur.

11. Dispositif de capteurs selon l'une des revendications précédentes, dans lequel la première fréquence d'évaluation et/ou la deuxième fréquence d'évaluation est supérieure ou égale à 0 Hz ; et/ou
dans lequel l'unité d'évaluation inclut une FFT ; ou
dans lequel l'unité d'évaluation inclut une FFT, et la première mesure est effectuée à une première fréquence d'évaluation égale à 0 Hz et/ou la deuxième mesure est effectuée à une deuxième fréquence d'évaluation supérieure à 0 Hz, égale à 0 Hz, ou à 1-OMEGA, 2-OMEGA ou 3-OMEGA.

12. Capteur de débit comprenant un dispositif de capteurs selon l'une des revendications précédentes, dans lequel le capteur de débit est conçu pour déterminer un débit en tenant compte de la conductivité thermique (k) et de la capacité thermique volumique (cv) déterminées.

13. Capteur de pression incluant un dispositif de capteurs selon l'une des revendications 1 à 11, dans lequel le capteur de pression est conçu pour déterminer la pression en tenant compte de la capacité thermique volumique (cv) et de la conductivité thermique (k).

14. Procédé d'évaluation, au moyen de l'unité d'évaluation (50), d'un dispositif de capteurs selon l'une des revendications 1 à 11, comprenant le fait de :
exciter le capteur (10) au moyen d'une fréquence d'excitation, une première fréquence d'excitation ou une première fréquence d'évaluation étant utilisée pour une première mesure (M1), et une deuxième fréquence d'excitation ou une deuxième fréquence d'évaluation étant utilisée pour une deuxième mesure (M2), la première fréquence d'excitation étant différente de la deuxième fréquence d'excitation ou la première fréquence d'évaluation étant différente de la deuxième fréquence d'évaluation ; et
déterminer, sur la base de la première mesure (M1), une conductivité thermique (k) du gaz environnant et, sur la base de la deuxième mesure (M2), une capacité thermique volumique (cv) du gaz environnant ;
**caractérisé en ce que** la première fréquence d'excitation est inférieure d'au moins un facteur 2 ou d'au moins un facteur 4 à la fréquence limite du capteur (10) et la deuxième fréquence d'excitation est supérieure d'au moins un facteur 2 ou d'au moins un facteur 4 à la fréquence limite du capteur (10) ; et/ou la première fréquence d'évaluation est inférieure d'au moins un facteur 2 ou d'au moins un facteur 4 à la fréquence limite du capteur (10) et la deuxième fréquence d'évaluation est supérieure d'au moins un facteur 2 ou d'au moins un facteur 4 à la fréquence limite du capteur (10).

15. Programme informatique destiné à mettre en œuvre le procédé selon la revendication 14, lorsque le procédé est exécuté sur l'unité d'évaluation (50).
